# EUROPEAN PATENT SPECIFICATION

(11) **EP 1 732 903 B1**
(45) Date of publication and mention of the grant of the patent: **18.02.2009**
(21) Application number: 05722275.4
(22) Date of filing: 29.03.2005
(51) Int. Cl.: C07D 249/08, A61K 31/4196, A61P 11/06, A61P 11/00, C07D 213/36, C07C 311/50

(54) **TRIAZOLONE DERIVATIVES AS MMP INHIBITORS FOR THE TREATMENT OF ASTHMA AND COPD**
TRIAZOLONDERIVATE ALS MMP-INHIBITOREN ZUR BEHANDLUNG VON ASTHMA UND COPD
DERIVES DE LA TRIAZOLONE UTILISES COMME INHIBITEURS DE LA MMP DANS LE TRAITEMENT DE L'ASTHME ET DE LA BPCO

(30) Priority: 30.03.2004 SE 0400850
(43) Date of publication of application: 20.12.2006
(73) Proprietor: AstraZeneca AB, 151 85 Södertälje (SE)
(72) Inventor: ERIKSSON, Anders, S-221 87 Lund (SE); LEPISTÖ, Matti, S-221 87 Lund (SE)
(86) International application number: PCT/SE2005/000448
(87) International publication number: WO 2005/095362

(56) References cited:
- EP-A1- 0 726 258
- EP-A1- 1 110 958
- WO-A1-00/74679
- WO-A1-01/46167
- WO-A1-03/078376
- WO-A1-03/089429
- WO-A1-03/092690
- XU Y. ET AL.: 'Desing and synthesis of a potent and selective triazolone-base' J.MED.CHEM. vol. 46, 2003, pages 5121 - 5124, XP002299289
- ROMINE J. ET AL.: '4,5-diphenyltriazol-3-ones: openers of large-conductante Ca2+activated potassium (maxi-K) channels.' J.MED.CHEM. vol. 45, 2002, pages 2942 - 2952, XP002989030
- BECKETT R.P.; WHITTAKER M.: 'Matrix metalloproteinase inhibitors 1998' EXP. OPIN. THER. PATENTS vol. 8, no. 3, 01 January 1998, ASHLEY PUBLICATIONS, GB, pages 259 - 282, XP002430631
- WHITTAKER M. ET AL: 'DESIGN AND THERAPEUTIC APPLICATION OF MATRIX METALLOPROTEINASE INHIBITORS' CHEM. REV. vol. 99, no. 9, 01 September 1999, ACS, WASHINGTON, DC, US, pages 2735 - 2776, XP000852443

## Description

The present invention relates to novel triazolone derivatives, processes for their preparation, pharmaceutical compositions containing them and their use in therapy.

Metalloproteinases are a superfamily ofproteinases (enzymes) whose numbers in recent years have increased dramatically. Based on structural and functional considerations these enzymes have been classified into families and subfamilies as described in N.M. Hooper (1994) FEBS Letters 354:1-6. Examples of metalloproteinases include the matrix metalloproteinases (MMPs) such as the collagenases (MMP1, MMP8, MMP13), the gelatinases (MMP2, MMP9), the stromelysins (MMP3, MMP10, MMP11), matrilysin (MMP7), metalloelastase (MMP12), enamelysin (MMP19), the MT-MMPs (MMP14, MMP15, MMP16, MMP17); the reprolysin or adamalysin or MDC family which includes the secretases and sheddases such as TNF converting enzymes (ADAM 10 and TACE); the astacin family which include enzymes such as procollagen processing proteinase (PCP); and other metalloproteinases such as aggrecanase, the endothelin converting enzyme family and the angiotensin converting enzyme family.

Metalloproteinases are believed to be important in a plethora of physiological disease processes that involve tissue remodelling such as embryonic development, bone formation and uterine remodelling during menstruation. This is based on the ability of the metalloproteinases to cleave a broad range of matrix substrates such as collagen, proteoglycan and fibronectin. Metalloproteinases are also believed to be important in the processing, or secretion, of biological important cell mediators, such as tumour necrosis factor (TNF); and the post translational proteolysis processing, or shedding, of biologically important membrane proteins, such as the low affinity IgE receptor CD23 (for a more complete list see N. M. Hooper et al., (1997) Biochem J. 321:265-279).

Metalloproteinases have been associated with many diseases or conditions. Inhibition of the activity of one or more metalloproteinases may well be of benefit in these diseases or conditions, for example: various inflammatory and allergic diseases such as, inflammation of the joint (especially rheumatoid arthritis, osteoarthritis and gout), inflammation of the gastro-intestinal tract (especially inflammatory bowel disease, ulcerative colitis and gastritis), inflammation of the skin (especially psoriasis, eczema, dermatitis); in tumour metastasis or invasion; in disease associated with uncontrolled degradation of the extracellular matrix such as osteoarthritis; in bone resorptive disease (such as osteoporosis and Paget's disease); in diseases associated with aberrant angiogenesis; the enhanced collagen remodelling associated with diabetes, periodontal disease (such as gingivitis), corneal ulceration, ulceration of the skin, post-operative conditions (such as colonic anastomosis) and dermal wound healing; demyelinating diseases of the central and peripheral nervous systems (such as multiple sclerosis); Alzheimer's disease; extracellular matrix remodelling observed in cardiovascular diseases such as restenosis and atheroscelerosis; asthma; rhinitis; and chronic obstructive pulmonary diseases (COPD).

MMP12, also known as macrophage elastase or metalloelastase, was initially cloned in the mouse by Shapiro et al [1992, Journal of Biological Chemistry 267: 4664] and in man by the same group in 1995. MMP12 is preferentially expressed in activated macrophages, and has been shown to be secreted from alveolar macrophages from smokers [Shapiro et al, 1993, Journal of Biological Chemistry, 268: 23824] as well as in foam cells in atherosclerotic lesions [Matsumoto et al, 1998, Am J Pathol 153: 109]. A mouse model of COPD is based on challenge of mice with cigarette smoke for six months, two cigarettes a day six days a week. Wild-type mice developed pulmonary emphysema after this treatment. When MMP12 knock-out mice were tested in this model they developed no significant emphysema, strongly indicating that MMP12 is a key enzyme in the COPD pathogenesis. The role of MMPs such as MMP12 in COPD (emphysema and bronchitis) is discussed in Anderson and Shinagawa, 1999, Current Opinion in Anti-inflammatory and Immunomodulatory Investigational Drugs 1(1) : 29-38. It was recently discovered that smoking increases macrophage infiltration and macrophage-derived MMP-12 expression in human carotid artery plaques Kangavari [Matetzky S, Fishbein MC et al., Circulation 102: 18, 36-39 Suppl. S, Oct 31, 2000].

MMP9 (Gelatinase B; 92kDa TypeIV Collagenase; 92kDa Gelatinase) is a secreted protein which was first purified, then cloned and sequenced, in 1989 [S.M. Wilhelm et al (1989) J. Biol Chem. 264 (29): 17213-17221; published erratum in J. Biol Chem. (1990) 265 (36): 22570]. A recent review of MMP9 provides an excellent source for detailed information and references on this protease: T.H. Vu & Z. Werb (1998) (In : Matrix Metalloproteinases. 1998. Edited by W.C. Parks & R.P. Mecham. pp 115 - 148. Academic Press. ISBN 0-12-545090-7). The following points are drawn from that review by T.H. Vu & Z. Werb (1998).

The expression of MMP9 is restricted normally to a few cell types, including trophoblasts, osteoclasts, neutrophils and macrophages. However, it's expression can be induced in these same cells and in other cell types by several mediators, including exposure of the cells to growth factors or cytokines. These are the same mediators often implicated in initiating an inflammatory response. As with other secreted MMPs, MMP9 is released as an inactive Pro-enzyme which is subsequently cleaved to form the enzymatically active enzyme. The proteases required for this activation *in vivo* are not known. The balance of active MMP9 versus inactive enzyme is further regulated *in vivo* by interaction with TIMP-1 (Tissue Inhibitor of Metalloproteinases -1), a naturally-occurring protein. TIMP-1 binds to the C-terminal region of MMP9, leading to inhibition of the catalytic domain of MMP9. The balance of induced expression of ProMMP9, cleavage of Pro- to active MMP9 and the presence of TIMP-1 combine to determine the amount of catalytically active MMP9 which is present at a local site. Proteolytically active MMP9 attacks substrates which include gelatin, elastin, and native Type IV and Type V collagens; it has no activity against native Type I collagen, proteoglycans or laminins.

There has been a growing body of data implicating roles for MMP9 in various physiological and pathological processes. Physiological roles include the invasion of embryonic trophoblasts through the uterine epithelium in the early stages of embryonic implantation; some role in the growth and development of bones; and migration of inflammatory cells from the vasculature into tissues.

MMP9 release, measured using enzyme immunoassay, was significantly enhanced in fluids and in AM supernantants from untreated asthmatics compared with those from other populations [Am. J. Resp. Cell & Mol. Biol., Nov 1997, 17(5):583-591]. Also, increased MMP9 expression has been observed in certain other pathological conditions, thereby implicating MMP9 in disease processes such as COPD, arthritis, tumour metastasis, Alzheimer's, Multiple Sclerosis, and plaque rupture in atherosclerosis leading to acute coronary conditions such as Myocardial Infarction.

A number of metalloproteinase inhibitors are known (see for example the reviews of MMP inhibitors by Beckett R.P. and Whittaker M., 1998, Exp. Opin. Ther. Patents, 8(3):259-282, and by Whittaker M. et al, 1999, Chemical Reviews 99(9):2735-2776).

We have now discovered a new class of compounds, namely triazolone derivatives, that are inhibitors of metalloproteinases and are of particular interest in inhibiting MMPs such as MMP12 and MMP9. The compounds of the present invention have beneficial potency, selectivity and/or pharmacokinetic properties. Certain compounds of the invention may also be useful as inhibitors of TACE and/or aggrecanase.

In accordance with the present invention, there is therefore provided a compound of formula (I) or a pharmaceutically acceptable salt or solvate thereof 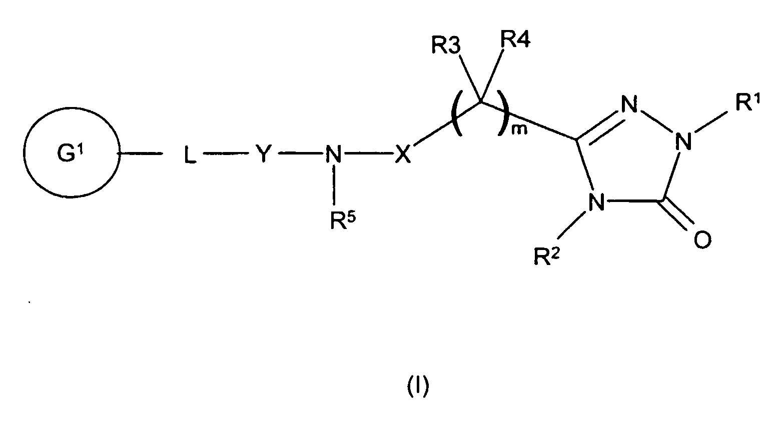 wherein
R¹ and R² independently represent H or C1 to 6 alkyl; said alkyl being optionally further substituted by an aryl ring or an aromatic heterocyclic ring containing 1 to 3 heteroatoms independently selected from O, S and N; said aromatic ring being optionally further substituted by halogen, CF₃, C1 to 4 alkyl or C1 to 4 alkoxy;
Each R³ and each R⁴ independently represents H or C1 to 6 alkyl; said alkyl being optionally further substituted by OH, C1 to 4 alkoxy, C1 to 4 alkylthio, amino, N-alkylamino or N,N-dialkylamino;
or R³ and R⁴ are bonded together so as to form a 3 to 7 membered ring; said ring optionally incorporating one heteroatom selected from O, S(O)q and N;
m represents an integer 1, 2 or 3;
X represents a group S(O), S(O)₂ or C(=O);
R⁵ represents H or C1 to 6 alkyl; said alkyl being optionally further substituted by halogen, OH or C1 to 6 alkoxy;
Y represents a direct bond;
or Y and R⁵ are bonded together such that the group -NR⁵Y- together represents a 4 to 7 membered saturated or partially unsaturated azacyclic ring; said azacyclic ring optionally incorporating one further heteroatom selected from O,-S(O)ₙ and N; said azacyclic ring being optionally benzo fused; said azacyclic ring being optionally substituted by C1 to 6 alkyl, C1 to 6 alkoxy or OH;
L represents a direct bond;
or L represents O, S(O)p, C(O), NR⁶, C(O)NR⁶, NR⁶C(O), C2 to 6 alkynyl, C2 to 6 alkenyl, C1 to 6 alkyl, C1 to 6 heteroalkyl or C3 to 6 heteroalkynyl; said alkyl, alkenyl or alkynyl group being optionally further substituted by halogen, OH or C1 to 6 alkoxy;
n, p and q independently represent an integer 0, 1 or 2;
G¹ represents a monocyclic, bicyclic, tricyclic or tetracyclic group comprising one, two, three or four ring structures each of up to 7 ring atoms; each ring structure being independently selected from cycloalkyl; cycloalkenyl; heterocycloalkyl; unsaturated heterocycloalkyl; aryl; or an aromatic heterocyclic ring containing 1 to 3 heteroatoms independently selected from O, S and N; with each ring structure being independently optionally substituted by one or more substituents independently selected from halogen, hydroxy, CHO, C1 to 6 alkyl, C1 to 6 alkoxy, halo-C1 to 6 alkoxy, amino, N-alkylamino, N,N-dialkylamino, alkylsulfonamino, C2 to 6 alkanoylamino, cyano, nitro, thiol, alkylthio, alkylsulfonyl, alkylaminosulfonyl, C2 to 6 alkanoyl, aminocarbonyl, N-alkylaminocarbonyl, N,N-amino-carbonyl;
wherein any alkyl radical within any substituent may itself be optionally substituted with one or more groups selected from halogen, hydroxy, C1 to 6 alkoxy,
halo-C1 to 6 alkoxy, amino, N-alkylamino, N,N-dialkylamino, N-alkylsulfonamino,
N-C2 to 6 alkanoylamino, cyano, nitro, thiol, alkylthio, alkylsulfonyl,
N-alkylaminosulfonyl, CHO, C2 to 6 alkanoyl, aminocarbonyl,
N-alkylaminocarbonyl, N,N-dialkylaminocarbonyl and carbamate;
and wherein any alkyl radical is a C1 to 6 alkyl radical;
and when G¹ is a bicyclic, tricyclic or tetracyclic group, each ring structure is independently joined to the next ring structure by a direct bond, by -O-, by C1-6 alkyl, by C-6 haloalkyl, by C1-6 heteroalkyl, by C2-6 alkenyl, by C2-6 alkynyl, by sulfone, by CO, by NR⁷CO, by CONR⁷ by NR⁷, by S, or by C(OH), or each ring structure is fused to the next ring structure;
R⁶ and R⁷ independently represent H or C1 to 6 alkyl;
and when the group -NR⁵Y- represents an azacyclic ring and L represents a direct bond, the group G¹ may also be spiro fused to the azacyclic ring;
and pharmaceutically acceptable salts thereof.

The compounds of formula (I) may exist in enantiomeric forms. It is to be understood that all enantiomers, diastereomers, racemates and mixtures thereof are included within the scope of the invention.

Compounds of formula (I) may also exist in various tautomeric forms. Thus, for example, the triazolone ring of compounds in which R¹ and R² each represent H can exist in the following tautomeric forms:

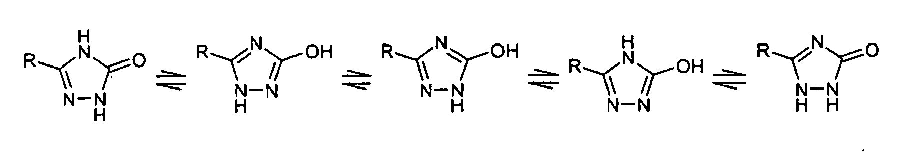

All possible tautomeric forms and mixtures thereof are included within the scope of the invention.

In one embodiment, X represents S(O)₂. In another embodiment, X represents C(=O).

In one embodiment, R¹ represents H. In one embodiment, R² represents H. In another embodiment, R¹ and R² each represent H.

In one embodiment, R³ and R⁴ independently represent H or C1 to 6 alkyl. In another embodiment, R³ and R⁴ each represent H.

In one embodiment, m represents the integer 1. In another embodiment, m represents the integer 2.

In one embodiment, R⁵ represents H or C1 to 6 alkyl. In another embodiment, R⁵ represents H.

In one embodiment, Y represents a direct bond.

In another embodiment, Y and R⁵ are bonded together such that the group NR⁵Y-together represents a 4 to 7 membered saturated or partially unsaturated azacyclic ring; said azacyclic ring optionally containing one further heteroatom selected from O, S(O)ₙ and N; said azacyclic ring being optionally benzo fused.

In another embodiment, Y and R⁵ are bonded together such that the group -NR⁵Y - together represents a 4 to 7 membered saturated or partially unsaturated azacyclic ring; said azacyclic ring optionally containing one further heteroatom selected from O, S(O)ₙ and N.

In another embodiment, Y and R⁵ are bonded together such that the group -NR⁵Y-together represents piperidinyl, 3,4-dehydropiperidinyl or piperazinyl.

In one embodiment, L represents a direct bond. In another embodiment, L represents O, C2 to 6 alkynyl, C1 to 6 alkyl, C1 to 6 heteroalkyl or C3 to 6 heteroalkynyl.

In one embodiment, G¹ represents an optionally substituted monocyclic or bicyclic ring structure. In another embodiment, G¹ represents an optionally substituted monocyclic ring structure. In another embodiment, G¹ represents an optionally substituted phenyl or heteroaryl ring. In another embodiment, G¹ represents an optionally substituted bicyclic ring structure. In another embodiment, G¹ represents an optionally substituted bicyclic ring structure in which each ring is independently phenyl or heteroaryl. In another embodiment, G¹ represents an optionally substituted bicyclic ring structure in which the two rings are either bonded directly to one another or are separated by an O atom. In another embodiment, G¹ represents an optionally substituted bicyclic ring structure in which each ring is independently phenyl or heteroaryl and the two rings are either bonded directly to one another or are separated by an O atom.

In one embodiment, X represents S(O)₂; R¹ and R² each represent H; R and R⁴ independently represent H or C1 to 6 alkyl; m represents the integer 1 or 2; R⁵ represents H and Y represents a direct bond; or Y and R⁵ are bonded together such that the group -NR⁵Y - together represents piperidinyl, 3,4-dehydropiperidinyl or piperazinyl; L represents a direct bond, O, C2 to 6 alkynyl or C1 to 6 alkyl; and G¹ represents an optionally substituted monocyclic or bicyclic ring structure.

In one embodiment, X represents S(O)₂; R¹ and R each represent H; R³ and R⁴ each represent H; m represents the integer 1; R⁵ represents H and Y represents a direct bond; or Y and R⁵ are bonded together such that the group -NR⁵Y- together represents piperidinyl, 3,4-dehydropiperidinyl or piperazinyl; L represents a direct bond, O, C2 alkynyl or C1 to 4 alkyl; and G¹ represents an optionally substituted monocyclic or bicyclic ring structure in which each ring is independently phenyl or heteroaryl; and when G1 represents a bicyclic ring structure the two rings are either bonded directly to one another or are separated by an O atom.

Unless otherwise indicated, the term "C1 to 6 alkyl" referred to herein denotes a straight or branched chain alkyl group having from 1 to 6 carbon atoms. Examples of such groups include methyl, ethyl, n-propyl, 1-propyl, n-butyl, i-butyl and t-butyl. The term "C1 to 4 alkyl" is to be interpreted analogously.

The two alkyl moieties in a dialkylamino group may be the same or different.

Unless otherwise indicated, the term "C2 to 6 alkenyl" referred to herein denotes a straight or branched chain alkyl group having from 2 to 6 carbon atoms incorporating at least one carbon-carbon double bond. Examples of such groups include ethenyl, propenyl and butenyl.

Unless otherwise indicated, the term "C2 to 6 alkynyl" referred to herein denotes a straight or branched chain alkyl group having from 2 to 6 carbon atoms incorporating at least one carbon-carbon triple bond. Examples of such groups include ethynyl, propynyl, and butynyl.

Unless otherwise indicated, the term "C1 to 6 alkoxy" referred to herein denotes a straight or branched chain alkyl group having from 1 to 6 carbon atoms bonded to a molecule via an oxygen atom. Examples of such groups include methoxy, ethoxy, n-propoxy, i-propoxy and t-butoxy. The term "C1 to 6 alkylthio" is to be interpreted analogously but with bonding being via a sulphur atom. The terms "C1 to 4 alkoxy" and "C1 to 4 alkylthio" are to be interpreted analogously.

Unless otherwise indicated, the term "halogen" referred to herein denotes fluoro, chloro, bromo and iodo.

Unless otherwise indicated, the term "C1 to 6 heteroalkyl" referred to herein denotes a straight or branched chain alkyl group having from 1 to 6 carbon atoms and incorporating one or more heteroatoms selected independently from O, S(O)n and N. Examples of such groups include -O- (CH₂)₃-, -CH₂CH₂OCH₂-, -CH₂CH₂SCH₂CH₂-, -CH₂CH₂OCH₂CH₂OCH₂-. The term "C3 to 6 heteroalkynyl" is to be interpreted analogously and would include such groups as -C≡C-CH₂-O-.

Examples of a "C1 to 6 haloalkyl or halo-C1 to 6 alkoxy" include CH₂F, CHF₂, CF₃, CF₃CF₂, CF₃CH₂, CH₂FCH₂, CH₃CF₂, CF₃CH₂CH₂, OCF₃ and OCH₂CF₃.

Unless otherwise indicated, the term "C2 to 6 alkanoyl" referred to herein denotes a straight or branched chain alkyl group having from 1 to 5 carbon atoms bonded to a molecule via a carbonyl (C=O) group. Examples of such groups include acetyl, propionyl and pivaloyl.

Examples of a 4 to 7 membered saturated or partially unsaturated azacyclic ring; optionally incorporating one further heteroatom selected from O, S(O)ₙ or N; and optionally being benzo fused; include pyrrolidine, piperidine, 3,4-dehydropiperidine, tetrahydroquinoline, tetrahydrosioquinoline, piperazine, morpholine and perhydroazepine.

Examples of an aromatic heterocyclic ring of up to 7 ring atoms containing 1 to 3 heteroatoms independently selected from O, S and N include furan, thiophene, pyrrole, pyridine, thiazole, imidazole, oxazole, isoxazole, pyrazole, triazole, oxadiazole, thiadiazole, pyrazine, pyridazine and pyrimidine.

Examples of a cycloalkyl or cycloalkenyl ring containing up to 7 ring atoms include cyclopropyl, cyclobutyl, cyclopentyl, cyclopentenyl, cyclohexyl and cyclohexenyl.

Examples of a heterocycloalkyl or unsaturated heterocycloalkyl ring containing up to 7 ring atoms include pyrrolidine, tetrahydrofuran, dioxane, dioxolane, thiane, piperidine, 3,4-dehydropiperidine, piperazine, morpholine, thiomorpholine and perhydroazepine.

Examples of an aryl group include phenyl and naphthyl.

Examples of compounds wherein the group -NR⁵Y- represents an azacyclic ring and L represents a direct bond and the group G¹ is spiro fused to the azacyclic ring include structures such as:

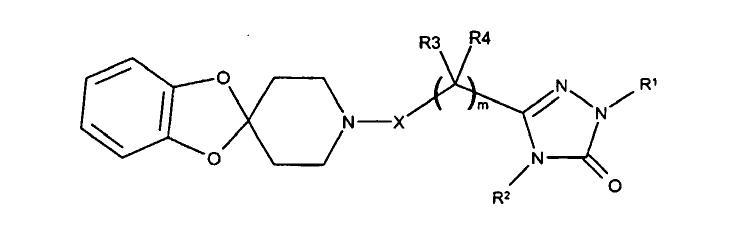

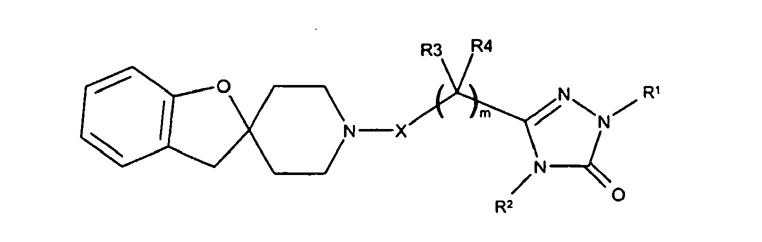

Specific examples of the molecular fragment 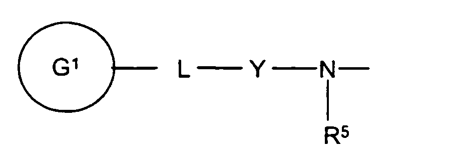 include 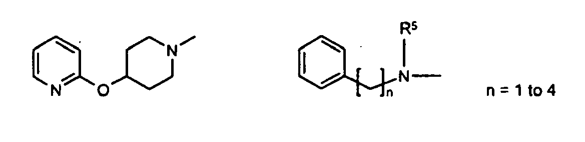 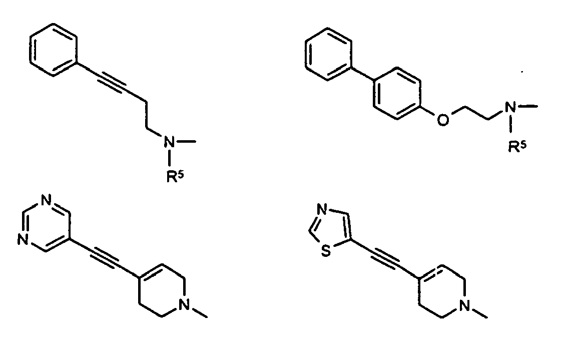 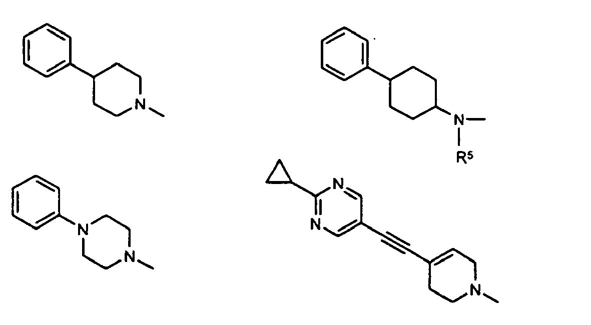 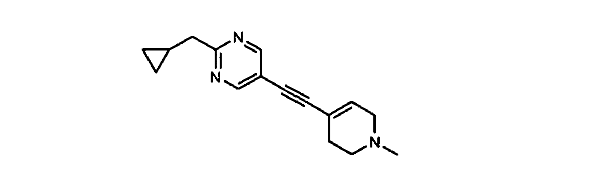 and corresponding structures in which the various rings are optionally substituted.

Specific examples of fused bicyclic ring systems include quinolinyl, isoquinolinyl, indolyl, tetrahydroisoquinolinyl, benzofuranyl, benzothienyl, quinazolinyl, phthalazinyl, dihydrobenzofuranyl, naphthyl and dihydroindolyl. Preferred bicyclic ring systems include quinolinyl, isoquinolinyl, tetrahydroisoquinolinyl, naphthyl, benzofuranyl and benzothienyl.

It will be appreciated that the particular substituents and number of substituents in the compounds of the invention are selected so as to avoid sterically undesirable combinations.

Examples of compounds of the invention include:
5-[({4-[(5-chloropyridin-2-yl)oxy]piperidin-1-yl}sulfonyl)methyl]-2,4-dihydro-3H-1,2,4-triazol-3-one;
5-[2-({4-[(5-chloropyridin-2-yl)oxy]piperidin-1-yl}sulfonyl)ethyl]-2,4-dihydro-3H-1,2,4-triazol-3-one;
5-[3-({4-[(5-chloropyridin-2-yl)oxy]piperidin-1-yl} sulfonyl)propyl]-2,4-dihydro-3H-1,2,4-triazol-3-one;
5-({[4-(4-chlorophenyl)piperazin-1-yl]sulfonyl}methyl)-2,4-dihydro-3H-1,2,4-triazol-3-one;
5-({[4-[(2-methoxypyrimidin-5-yl)ethynyl]-3,6-dihydropyridin-1(2H)-yl]sulfonyl}methyl)-2,4-dihydro-3H-1,2,4-triazol-3-one;
5-({[4-{[2-(trifluoromethyl)pyrimidin-5-yl]ethynyl}-3,6-dihydropyridin-1(2H)-yl]sulfonyl}methyl)-2,4-dihydro-3H-1,2,4-triazol-3-one;
5-({[4-[(2-cyclopropylpyrimidin-5-yl)ethynyl]-3,6-dihydropyridin-1 (2H)-yl]sulfonyl}methyl)-2,4-dihydro-3H-1,2,4-triazol-3-one;
5-({[4-(4-chlorophenyl)piperidin-1-yl]sulfonyl} methyl)-2,4-dihydro-3H-1,2,4-triazol-3-one;
N-benzyl-1-(5-oxo-4,5-dihydro-1H-1,2,4-triazol-3-yl)methanesulfonamide;
1-(5-oxo-4,5-dihydro-1 H-1,2,4-triazol-3-yl)-N-(2-phenylethyl)methanesulfonamide;
5-(2-{[4-(4-chlorophenyl)piperidin-1-yl]sulfonyl}ethyl)-2,4-dihydro-3H-1,2,4-triazol-3-one;
5-(2- {[4-(4-chlorophenyl)piperazin-l-yl]sulfonyl}ethyl)-2,4-dihydro-3H-1,2,4-triazol-3-one;
5-(3- {[4-(4-chlorophenyl)piperidin-1-yl]sulfonyl}propyl)-2,4-dihydro-3H-1,2,4-triazol-3-one;
5-(3- {[4-(4-chlorophenyl)piperazin-1-yl]sulfonyl}propyl)-2,4-dihydro-3H-1,2,4-triazol-3-one;
and pharmaceutically acceptable salts and solvates thereof.

Each exemplified compound represents a particular and independent aspect of the invention.

The compounds of formula (I) may exist in enantiomeric forms. Therefore, all enantiomers, diastereomers, racemates and mixtures thereof are included within the scope of the invention. The various optical isomers may be isolated by separation of a racemic mixture of the compounds using conventional techniques, for example, fractional crystallisation, or HPLC. Alternatively the optical isomers may be obtained by asymmetric synthesis, or by synthesis from optically active starting materials.

Where optically isomers exist in the compounds of the invention, we disclose all individual optically active forms and combinations of these as individual specific embodiments of the invention, as well as their corresponding racemates.

Where tautomers exist in the compounds of the invention, we disclose all individual tautomeric forms and combinations of these as individual specific embodiments of the invention.

The present invention includes compounds of formula (I) in the form of salts. Suitable salts include those formed with organic or inorganic acids or organic or inorganic bases. Such salts will normally be pharmaceutically acceptable salts although non-pharmaceutically acceptable salts may be of utility in the preparation and purification of particular compounds. Such salts include acid addition salts such as hydrochloride, hydrobromide, citrate, tosylate and maleate salts and salts formed with phosphoric acid or sulphuric acid. In another aspect suitable salts are base salts such as an alkali metal salt, for example, sodium or potassium, an alkaline earth metal salt, for example, calcium or magnesium, or an organic amine salt, for example, triethylamine. Examples of solvates include hydrates.

Salts of compounds of formula (I) may be formed by reacting the free base or another salt thereof with one or more equivalents of an appropriate acid or base.

The compounds of formula (I) are useful because they possess pharmacological acivity in animals and are thus potentially useful as pharmaceuticals. In particular, the compounds of the invention are metalloproteinase inhibitors and may thus be used in the treatment of diseases or conditions mediated by MMP12 and/or MMP9 such as asthma, rhinitis, chronic obstructive pulmonary diseases (COPD), arthritis (such as rheumatoid arthritis and osteoarthritis), atherosclerosis and restenosis, cancer, invasion and metastasis, diseases involving tissue destruction, loosening of hip joint replacements, periodontal disease, fibrotic disease, infarction and heart disease, liver and renal fibrosis, endometriosis, diseases related to the weakening of the extracellular matrix, heart failure, aortic aneurysms, CNS related diseases such as Alzheimer's disease and Multiple Sclerosis (MS), and hematological disorders.

Accordingly, the present invention provides a compound of formula (I), or a pharmaceutically acceptable salt or solvate thereof, as hereinbefore defined for use in therapy.

In another aspect, the invention provides the use of a compound of formula (I), or a pharmaceutically acceptable salt or solvate thereof, as hereinbefore defined in the manufacture of a medicament for use in therapy.

In another aspect, the invention provides the use of a compound of formula (I), or a pharmaceutically acceptable salt or solvate thereof, as hereinbefore defined in the manufacture of a medicament for use in the treatment of diseases or conditions in which inhibition of MMP12 and/or MMP9 is beneficial.

In another aspect, the invention provides the use of a compound of formula (I), or a pharmaceutically acceptable salt or solvate thereof, as hereinbefore defined in the manufacture of a medicament for use in the treatment of inflammatory disease.

In another aspect, the invention provides the use of a compound of formula (I), or a pharmaceutically acceptable salt or solvate thereof, as hereinbefore defined in the manufacture of a medicament for use in the treatment of an obstructive airways disease such as asthma or COPD.

In the context of the present specification, the term "therapy" also includes "prophylaxis" unless there are specific indications to the contrary. The terms "therapeutic" and "therapeutically" should be construed accordingly.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above-mentioned therapeutic uses the dosage administered will, of course, vary with the compound employed, the mode of administration, the treatment desired and the disorder to be treated. The daily dosage of the compound of formula (I)/salt/solvate (active ingredient) may be in the range from 0.001 mg/kg to 75 mg/kg, in particular from 0.5 mg/kg to 30 mg/kg. This daily dose may be given in divided doses as necessary-Typically unit dosage forms will contain about 1 mg to 500 mg of a compound of this invention.

The compounds of formula (I) and pharmaceutically acceptable salts and solvates thereof may be used on their own but will generally be administered in the form of a pharmaceutical composition in which the formula (I) compound/s salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.10 to 70 %w, of active ingredient, and, from 1 to 99.95 %w, more preferably from 30 to 99.90 %w, of a pharmaceutically acceptable adjuvant, diluent or carrier, all percentages by weight being based on total composition. Conventional procedures for the selection and preparation of suitable pharmaceutical formulations are described in, for example, "Pharmaceuticals - The Science of Dosage Form Designs", M. E. Aulton, Churchill Livingstone, 1988.

Thus, the present invention also provides a pharmaceutical composition comprising a compound of formula (I) or a pharmaceutically acceptable salt or solvate thereof as hereinbefore defined in association with a pharmaceutically acceptable adjuvant, diluent or carrier.

The invention further provides a process for the preparation of a pharmaceutical composition of the invention which comprises mixing a compound of formula (I) or a pharmaceutically acceptable salt or solvate thereof as hereinbefore defined with a pharmaceutically acceptable adjuvant, diluent or carrier.

The pharmaceutical compositions of this invention may be administered in standard manner for the disease or condition that it is desired to treat, for example by oral, topical, parenteral, buccal, nasal, vaginal or rectal administration or by inhalation. For these purposes the compounds of this invention may be formulated by means known in the art into the form of, for example, tablets, capsules, aqueous or oily solutions, suspensions, emulsions, creams, ointments, gels, nasal sprays, suppositories, finely divided powders or aerosols for inhalation, and for parenteral use (including intravenous, intramuscular or infusion) sterile aqueous or oily solutions or suspensions or sterile emulsions.

In addition to the compounds of the present invention the pharmaceutical composition of this invention may also contain, or be co-administered (simultaneously or sequentially) with, one or more pharmacological agents of value in treating one or more diseases or conditions referred to hereinabove such as "Symbicort" (trade mark) product.

The present invention further provides a process for the preparation of a compound of formula (I) or a pharmaceutically acceptable salt or solvate thereof as defined above which comprises:
reaction of a compound of formula (II) 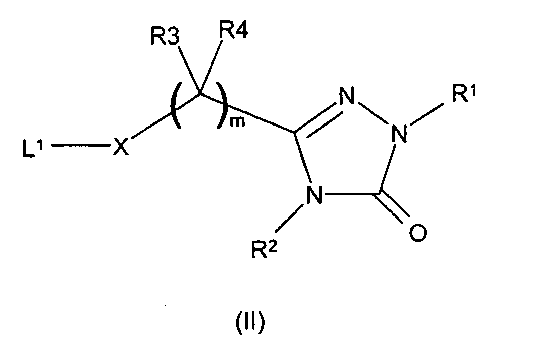
wherein R¹, R², R³, R⁴, X and m are as defined in formula (I) and L¹ represents a leaving group, with a compound of formula (III) 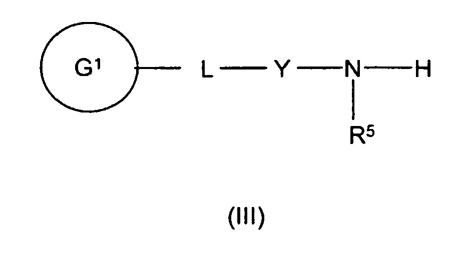 wherein G¹, L, Y and R⁵ are as defined in formula (I)
and optionally thereafter forming a pharmaceutically acceptable salt or solvate.

In the above process, suitable leaving groups L¹ include halo, particularly chloro. The reaction is preferably performed in a suitable solvent optionally in the presence of an added base for a suitable period of time, typically 1 to 24 h, at ambient to reflux temperature. Preferably, solvents such as pyridine, dimethylformamide, tetrahydrofuran, acetonitrile or dichloromethane are used. When used the added base may be an organic base such as triethylamine, diisopropyethylamine, N-methylmorpholine or pyridine, or an inorganic base such as an alkali metal carbonate. The reaction is typically conducted at ambient temperature for 2 to 16 h, or until completion of the reaction has been achieved, as determined by chromatographic or spectroscopic methods. Reactions of sulfonyl halides and acyl halides with various primary and secondary amines are well known in the literature, and the variations of the conditions will be evident for those skilled in the art.

Compounds of formula (II) wherein X represents S(O)₂ and L¹ represents chloro are conveniently prepared by oxidative chlorination of alkyl or benzyl thioethers of formula (IV) (Griffith, O.: J. Biol. Chem., 1983, 258, 3, 1591). 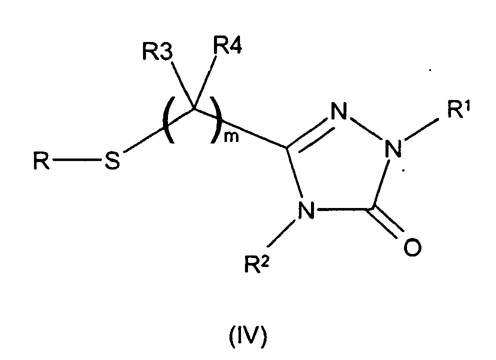 wherein R represents a C1 to 6 alkyl or benzyl residue. Typically R represents unsubstituted benzyl (Ph-CH₂) or tert-butyl.

Compounds of formula (IV) may be prepared by reacting a compound of formula (V) in which L² is a leaving group, for example, halo or a sulfonate ester, 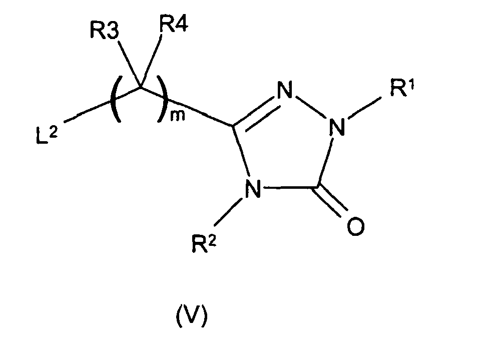 with an alkyl or benzyl thiol, R-SH. The reactions are preferably performed in the presence of a base such as diethylisopropylamine or caesium carbonate and in the presence of a suitable solvent, for example, DMF.

Compounds of formula (V) may be prepared from, for example, corresponding carboxylic acids and derivatives thereof, using, for example, methods that will be readily apparent to the man skilled in the art. See, for example, B. George et al, J. Org. Chem. 1976, 41(20), 3233; H-C Huang et al, J. Med. Chem. 1993, 36(15), 2172; C.J. Crowden et al, Tetrahedron Letters, 2000, 41, 8661; Y. Xu et al, J. Med. Chem. 2003, 46(24), 5121).

It will be appreciated by those skilled in the art that in the processes of the present invention certain potentially reactive functional groups such as hydroxyl or amino groups in the starting reagents or intermediate compounds may need to be protected by suitable protecting groups. Thus, the preparation of the compounds of the invention may involve, at various stages, the addition and removal of one or more protecting groups.

Suitable protecting groups and details of processes for adding and removing such groups are described in 'Protective Groups in Organic Chemistry', edited by J.W.F. McOmie, Plenum Press (1973) and 'Protective Groups in Organic Synthesis', 3rd edition, T.W. Greene and P.G.M. Wuts, Wiley-Interscience (1999).

The compounds of the invention and intermediates thereto may be isolated from their reaction mixtures and, if necessary further purified, by using standard techniques.

The present invention will now be further explained by reference to the following illustrative examples.

In the Examples, ¹H-NMR and ¹³C-NMR spectra were recorded on either a Varian ^{Unity}*Inova* 400MHz or Varian *Mercury*-VX 300MHz instrument. The central solvent peak of dimethylsulfoxide-d₆ (δ_{H} 2.50 ppm), tetrahydrofuran-d₈ (δ_{H} 3.58, 1.73 ppm), chloroform-d (δ_{H} 7.27 ppm) or methanol-d₄ (δ_{H} 3.31 ppm) were used as internal references.

The following method was used for LC/MS analysis:
Instrument Agilent 1100; Column Waters Symmetry 2.1 x 30 mm; Mass APCI; Flow rate 0.7 mL/min; Wavelength 254 or 220 nm; Solvent A: water + 0.1% TFA; Solvent B: acetonitrile + 0.1% TFA ; Gradient 15-95%/B 2.7 min, 95% B 0.3 min.
Column chromatography was carried out using silica gel (0.040-0.063 mm, Merck).

All solvents and commercial reagents were laboratory grade and used as received. Non-commercially available reagents were synthesised using known literature procedures. Abbreviations used include:
- DIEA: N,N-diisopropylethylamine;
- DCM: dichloromethane;
- THF: tetrahydrofuran;
- THF-D8: deuterated tetrahydrofuran;
- AcOH: acetic acid;
- MeCN: acetonitrile;
- DMF: N,N-dimethylformamide;
- EtOAc: ethyl acetate;
- DMSO: dimethyl sulfoxide;
- DMSO-D6: deuterated dimethyl sulfoxide;
- Et₂O: diethylether;
- Et₂NH: diethylamine;
- TFA: trifluoroacetic acid;
- IPA: 2-propanol;
- LC/MS: liquid chromatography/mass spectrometry;
- TLC: thin layer chromatography;

### Example 1

### 5-[({14-[(5-Chloropyridin-2-yl)oxy]piperidin-1-yl}sulfonyl)methyl]-2,4-dihydro-3H-1,2,4-triazol-3-one

### a) 5-[(Benzylthio)methyl]-2,4-dihydro-3H-1,2,4-triazol-3-one

Benzylmercaptan (1.75 mL; 14.9 mmol) was dissolved in DMF (20 mL) and solid K₂CO₃ (2.35 g; 17 mmol) was added. To the resulting slurry was added a solution of 5-(chloromethyl)-2,4-dihydro-3H-1,2,4-triazol-3-one (2.0 g; 15 mmol) in DMF (12 mL), prepared by a literature procedure (C. J. Cowden et. al., Tetrahedron Letters 41 (2000) 8661-8664). The reaction mixture was stirred at room temperature for 20.5 h. Water (80 mL) was added and a thick slurry was formed. The solid product was collected by filtration and washed with water. The remaining filtrate and wash liquid still contained product and was extracted four times with EtOAc, and the organic phase was then washed with water (twice), brine (twice) and dried (Na₂SO₄). Evaporation of solvents gave another crop of crude product. The combined solid materials were suspended in toluene and evaporated to remove water residues. The crude product was then suspended in a boiling mixture of EtOAc/heptane (1:4) and allowed to cool before the solid product was collected by filtration. The subtitle compound was obtained as a colourless solid (2.03 g; 61% yield).
APCI-MS m/z: 222.1 [MH⁺].
¹H-NMR (DMSO-D6): δ 11.35 (1H, vbrs), 11.26 (1H, brs), 7.37-7.21 (5H, m), 3.72 (2H, s), 3.36 (2H, s) ppm.
¹³C-NMR (DMSO-D6): δ 156.09, 144.75, 137.66, 128.83, 128.23, 126.79, 34.75, 25.80 ppm.

### b) (5-Oxo-4,5-dihydro-1H-1,2,4-triazol-3-yl)methanesulfonyl chloride

5-[(Benzylthio)methyl]-2,4-dihydro-3H-1,2,4-triazol-3-one (0.5 g; 2.26 mmol) was dissolved in AcOH (18 mL) and water (2 mL). The solution was cooled on a ice-bath and Cl₂ gas was slowly bubbled through the solution for 5 min. The green-yellow solution was stirred for 10 min while reaching room temperature and argon gas was bubbled through the solution to remove excess Cl₂. The clear solution was evaporated to leave an oil which was re-suspended in toluene and evaporated. This process was repeated one more time. The crude product of (5-oxo-4,5-dihydro-1H-1,2,4-triazol-3-yl)methanesulfonyl chloride was obtained as a sticky oil still containing benzyl acetate and solvent residues as impurities. This material was dissolved in THF and used directly without further purification.
A sample for analytical purposes was obtained by triturating the crude material with isohexane, CHCl₃ and Et₂O in that order. After drying under reduced pressure the subtitle compound was obtained as a slightly yellow solid.
¹H-NMR (THF-D8): δ 10.93 (1.4H, vbrs, NH), 5.21 (2H, s, CH₂), 4.80-3.65 (0.9H, vbrs, H₂O + NH) ppm.
The reactivity of the sulfonyl chloride was confirmed by its reaction with Et₂NH to give the expected N,N-diethyl-1-(5-oxo-4,5-dihydro-1H-1,2,4-triazol-3-yl)methanesulfonamide.
APCI-MS m/z: 235.1 [MH⁺].

### c) 5-[({4-[(5-Chloropyridin-2-yl)oxy]piperidin-1-yl}sulfonyl)methyl]-2,4-dihydro-3H-1,2,4-triazol-3-one

5-Chloro-2-(piperldin-4-yloxy)pyridine (180 mg; 0.85 mmol) and DIEA (145 ul; 0.85 mmol) were dissolved in THF (3 mL), and a THF solution of crude (5-oxo-4,5-dihydro-1H-1,2,4-triazol-3-yl)methanesulfonyl chloride (approximately 0.56 mmol) was added. The reaction was stirred at room temperature for 1.5 h. Solvent was removed by evaporation and the residue was partitioned between EtOAc and 5% aqueous NaHSO₄ and separated. The water phase was extracted one more time with EtOAc and the combined organic phases were washed with brine and evaporated. The crude product was purified on a preparative HPLC system using a KROMASIL KR-100-7-C18, 250 x 50.8 mm column. A gradient of 20-90% MeCN/water plus 0.1% TFA was used with UV 220 nm for detection. The fractions that according to LC/MS contained the product were evaporated until a slurry was formed and the residual water was removed by freeze drying to leave crude product (40 mg). This material was further purified using a semi-prep HPLC system, KROMASIL 100-5-C18, 250 x 20 mm column, UV 220 nm, and a 80 min gradient of 25-27% MeCN/water plus 50 mM NH₄OAc. Freeze drying gave the title compound as a colourless solid (16 mg; 7.6% yield).
APCI-MS m/z: 374.2 [MH⁺].
¹H-NMR (DMSO-D6): δ 11.61 (1H, brs), 11.57 (1H, vbrs), 8.20 (1H, d), 7.81 (1H, dd), 6.87 (1H, d), 5.08 (1H, m), 4.32 (2H, s), 3.49-3.39 (2H, m), 3.23-3.13 (2H, m), 2.06-1.94 (2H, m), 1.77-1.64 (2H, m) ppm.
¹³C-NMR (DMSO-D6): δ 160.79, 155.69, 144.73, 139.14, 137.74, 123.22, 112.74, 69.57, 47.47, 42.80, 30.08 ppm.

### Example 2

### 5-[2-({4-[(5-Chloropyridin-2-yl)oxy]piperidin-1-yl)}sulfonyl)ethyl]-2,4-dihydro-3H-1,2,4-triazol-3-one

### a) 5-[2-(Benzylthio)ethyl]-2,4-dihydro-3H-1,2,4-triazol-3-one

3-(Benzylthio)propanoic acid (1.0 g; 5.1 mmol) was dissolved in THF (10 mL). DMF (100 uL) was added followed by dropwise addition of (COCl)₂ (0.45 mL; 5.2 mmol). After 1 h, a sample for LC was quenched with Et₂NH, showing that approximately 40% starting material remained. More (COCl)₂ (0.12 mL; 1.4 mmol) was added and the reaction mixture was stirred at room temperature for 2.5 h. A sample for LC was quenched as before with Et₂NH and showed that all starting material had been consumed.
The slightly yellow solution of 3-(benzylthio)propanoyl chloride was added to a pre-cooled solution of semicarbazide hydrochloride (0.95 g; 8.5 mmol) and NaOH (0.83 g; 20.8 mmol) in THF (10 mL) and water (2 mL). The slightly acidic (pH 5) solution was neutralised with a few drops of aqueous NaOH to pH 7. The reaction was allowed to reach room temperature and left overnight. A sample was withdrawn for LC/MS analysis and
APCI-MS m/z: 254.0 [MH⁺] for the intermediate
2-[3-(benzylthio)propanoyl]hydrazinecarboxamide was found as the major product. To the solution was added 2M aqueous NaOH (30 mL) and the mixture was heated to reflux for 23 h. The reaction mixture was allowed to reach room temperature and acidified using conc. HCl, extracted twice with EtOAc and the organic phase was dried (Na₂SO₄), filtered and evaporated to give crude product (1.08 g). This material was purified using flash chromatography using Si-60 gel and a solvent gradient of 0-10% IPA/DCM. The fractions containing the product were evaporated to give the subtitle compound as a colourless solid (0.34 g; 28%).
TLC (Si-60, DCM +10% IPA): R_{f} 0.4.
APCI-MS m/z: 236.1 [MH⁺].
¹H-NMR (DMSO-D6): δ 11.18 (1H, s), 11.13 (1H, s), 7.36-7.19 (5H, m), 3.75 (2H, s), 2.70-2.57 (4H, m) ppm.

### b) 2-(5-Oxo-4,5-dihydro-1H-1,2,4-triazol-3-yl)ethanesulfonyl chloride

5-[2-(Benzylthio)ethyl]-2,4-dihydro-3H-1,2,4-triazol-3-one (0.3 g; 1.27 mmol) was dissolved in AcOH (18 mL) and water (2 mL). The solution was cooled on an ice/water bath and Cl₂ (g) was slowly bubbled through the stirred solution. When the solution turned greenish yellow the introduction of chlorine was stopped. The cold bath was removed and the mixture was stirred for 10 min. Argon (g) was passed through the solution until it became colourless. The clear solution was freeze dried to give the subtitle compound (0.26 g; 97%) as a colourless solid.
¹H-NMR (THF-D8): δ 10.69 (1H, vbrs), 10.57 (1H, brs), 4.29 (2H, m), 3.15 (2H, m) ppm.

### c) 5-[2-({4-[(5-Chloropyridin-2-yl)oxy)piperidin-1-yl}sulfonyl)ethyl]ethyl]-2,4-dihydro-3H-1,2,4-triazol-3-one

5-Chloro-2-(piperidin-4-yloxy)pyridine (100 mg; 0.47 mmol) and DIEA (80 uL; 0.47 mmol) were dissolved in THF (3 mL). A solution of 2-(5-oxo-4,5-dihydro-1H-1,2,4-triazol-3-yl)ethanesulfonyl chloride (65 mg; 0.31 mmol) in THF (4 mL) was added dropwise at room temperature. The reaction was stirred for 1 h before the solvents were removed by evaporation. The residual material was purified using a preparative HPLC system, column Kromasil, KR-100-7-C18, 250 x 50.8 mm. A 40 minute gradient of 20-90% MeCN/water plus 0.1% TFA was used, and UV 220 nm for detection. Fractions containing the desired product were collected. Evaporation of the solvents gave a slurry from which the residual water was removed by freeze drying to give the title compound (90 mg; 74%) as a colourless solid.
APCI-MS m/z: 388.1 [MH⁺].
¹H-NMR (DMSO-D6): δ 11.25 (1H, s), 11.24 (1H, s), 8.20 (1H, d), 7.81 (1H, dd), 6.88 (1H, d), 5.11 (1H, m), 3.47-3.39 (2H, m), 3.39 (2H, t), 3.23-3.14 (2H, m), 2.81 (2H, t), 2.06-1.96 (2H, m), 1.77-1.66 (2H, m) ppm.
¹³C-NMR (DMSO-D6): δ 160.80, 155.86, 144.68, 144.20, 139.11, 123.19, 112.75, 69.58, 45.20, 42.49, 30.15, 20.87 ppm.

### Example 3

### 5[3-({4-[(5-Chloropyridin-2-yl)oxy]piperidin-1-yl}-sulfonyl)propyl]-2,4-dihydro-3H-1,2,4-triazol-3-one

### a) 5-(3-Bromopropyl)-2,4-dihydro-3H-1,2,4-triazol-3-one

This was prepared in a similar way to that described for 5-(chloromethyl)-2,4-dihydro-3H-1,2,4-triazol-3-one (C. J. Cowden et. al., Tetrahedron Letters 41 (2000) 8661-8664). Trimethyl 4-bromo-orthobutyrate (5 g; 22 mmol) and semicarbazide hydrochloride (1.12 g; 10 mmol) were stirred in MeOH for 20 h at room temperature. Evaporation of the solvents gave an oily residue that was treated with toluene and evaporated to remove MeOH residues, at which time a precipitate started to form in the toluene solution. The slurry was cooled on dry-ice and the solid material was removed by filtration and washed with toluene. The solid material (1.79 g) was suspended in water and neutralized with 5% aqueous NaHCO₃. The product was then extracted into EtOAc, dried over Na₂SO₄, filtered and evaporated to give the subtitle compound (1.7 g; 82%) as a colourless solid.
APCI-MS m/z: 206.0 and 208.0 [MH⁺].
¹H-NMR (DMSO-D6): δ 11.19 (1H, s), 11.11 (1H, s), 3.56 (2H, t), 2.51 (2H, t), 2.09 (2H, quintet) ppm.

### b) 5-[3-(Benzylthio)propyl]-2,4-dihydro-3H-1,2,4-triazol-3-one

Benzylmercaptan (0.9 mL; 7.7 mmol) was dissolved in DMF (10 mL) and K₂CO₃ (1.15 g; 8.3 mmol) was added. 5-(3-Bromopropyl)-2,4-dihydro-3H-1,2,4-triazol-3-one (1.6 g; 7.8 mmol) dissolved in DMF (6 mL) was added and the slurry was stirred for 21 h at room temperature. Water (40 mL) was added and an opaque solution was formed which was extracted four times with EtOAc. The organic phase was washed with water (twice) and brine, dried over Na₂SO₄, filtered and the solvent removed by evaporation. The residual colourless solid was re-dissolved in hot EtOAc (50 mL) and while stirring heptane (150 to 200 mL) was added to precipitate the desired product. After the slurry reached room temperature the solid was collected by filtration and washed with heptane, dried under reduced pressure at + 50 °C for 13 h to constant weight to give the subtitle compound (0.7 g; 36%) as a colourless solid.
APCI-MS m/z: 250.1 [MH⁺].
¹H-NMR (DMSO-D6): δ 11.16 (1H, s), 11.07 (1H, s), 7.34-7.20 (5H, m), 3.72 (2H, s), 2.43 (2H, t), 2.39 (2H, t), 1.81 (2H, quintet) ppm.
¹³-C-NMR (DMSO-D6): δ 156.01, 146.46, 138.40, 128.64, 128.16, 126.58, 34.69, 29.61, 25.60, 25.19 ppm.

### c) 3-(5-Oxo-4,5-dihydro-1H-1,2,4-triazol-3-yl)propane-1-sulfonyl chloride

5-[3-(Benzylthio)propyl]-2,4-dihydro-3H-1,2,4-triazol-3-one (0.5 g; 2.0 mmol) was dissolved in AcOH (18 mL) and water (2 mL). The solution was cooled on an ice/water bath and Cl₂ (g) was bubbled through the solution until a yellow green solution was obtained. The reaction mixture was stirred for 10 min and then the cold bath was removed. Argon (g) was bubbled through the solution until a clear colourless solution was obtained. Freeze drying gave the sub-title compound as an oil (0.63 g) containing benzyl acetate and solvent residues as major impurities. This material was dissolved in THF and used directly without further purification.
¹H-NMR (THF-D8): δ 12.00-9.20 (2H, baseline broad), 4.05 (2H, t), 2.71 (2H, t), 2.36 (2H, quintet) ppm.
The presence of reactive sulfonylchloride was confirmed by reacting a small sample of the obtained oil with 5-chloro-2-(piperidin-4-yloxy)pyridine to give the expected 5-[3-({4-[(5-chloropyridin-2-yl)oxy]piperidin-1-yl}sulfonyl)propyl]-2,4-dihydro-3H-1,2,4-triazol-3-one.
APCI-MS m/z: 402.1 [MH⁺].

### d) 5-[3-({4-[(5-Chloropyridin-2-yl)oxy]piperidin-1-yl}sulfonyl)propyl]-2,4-dihydro-3H-1,2,4-triazol-3-one

5-Chloro-2-(piperidin-4-yloxy)pyridine (0.16 g; 0.75 mmol) and DIEA (130 uL; 0.76 mmol) were dissolved in THF (3 mL). A THF solution (4 mL) containing crude 3-(5-oxo-4,5-dihydro-1H-1,2,4-triazol-3-yl)propane-1-sulfonyl chloride (maximum 0.5 mmol) was slowly added. The reaction was stirred overnight at room temperature and then the yellow slurry was evaporated. The residual material was suspended in MeCN/water and made acidic using a few drops of TFA. The insoluble product was filtered off and dried under reduced pressure. The title compound (137 mg; 68%) was obtained as a colourless solid shown to be 95% pure by HPLC.
APCI-MS m/z: 402.2 [MH⁺].
¹H-NMR (DMSO-D6): δ 11.22 (1H, s), 11.30 (1H, s), 8.20 (1H, d), 7.81 (1H, dd), 6.87 (1H, d), 5.11 (1H, m), 3.43 (2H, m), 3.21-3.08 (4H, m), 2.53 (2H, t), 2.08-1.92 (4H, m), 1.72 (2H, m) ppm.
¹³C-NMR (DMSO-D6): δ 160.81, 155.97, 146.04, 144.68, 139.10, 123.71, 112.72, 69.71, 47.42, 42.62, 30.17, 24.65, 19.86 ppm.

Following the general method of Example 1 but substituting the appropriate amine intermediate, and using 1 extra equivalent of the base DIEA if the amine salt was used, the compounds of Examples 4 to 10 were prepared:

### Example 4

### 5-({[4-(4-Chlorophenyl)piperazin-1-yl]sulfonyl}methyl)-2,4-dihydro-3H-1,2,4-triazol-3-one

APCI-MS m/z: 358.1 [MH⁺].
¹H-NMR (DMSO-D6): δ 11.61 (1H, s), 11.59 (1H, s), 7.26 (2H, d), 6.98 (2H, d), 4.36 (2H, s), 3.34-3.28 (4H, m), 3.22-3.16 (4H, m) ppm.

### Example 5

### 5-({[4-[(2-Methoxypyrimidin-5-yl)ethynyl]-3,6-dihydropyridin-1(2H)-yl]sulfonyl}methyl)-2,4-dihydro-3H-1,2,4-triazol-3-one

APCI-MS m/z: 377.1 [MH⁺].
¹H-NMR (DMSO-D6): δ 11.56 (2H, s), 8.72 (2H, s), 6.24 (1H, m), 4.37 (2H, s), 3.95 (3H, s), 3.90 (2H, m), 3.35 (2H, t), 2.35 (2H, m) ppm.
¹³C-NMR (DMSO-D6): δ 163.40, 161.15, 155.63, 137.62, 130.98, 117.81, 111.89, 93.41, 82.08, 54.98, 47.47, 44.54, 41.86, 28.86 ppm.
¹⁵N-¹H-correlated NMR showed a cross peak for two different ¹⁵N at 169.9 and 145.7 ppm to the same ¹H signal at 11.56 ppm.

### Example 6

### 5-({[4-{[2-(Trifluoromethyl)pyrimidin-5-yl]ethynyl}-3,6-dihydropyridin-1(2H)-yl]sulfonyl}methyl)-2,4-dihydro-3H-1,2,4-triazol-3-one

APCI-MS m/z: 415.0 [MH⁺].
¹H-NMR (DMSO-D6): δ 11.60 (2H, s), 9.16 (2H, s), 6.40 (1H, m), 4.38 (2H, s), 3.94 (2H, m), 3.37 (2H, t), 2.40 (2H, m) ppm.

### Example 7

### 5-({[4-[(2-Cyclopropylpyrimidin-5-yl)ethynyl]-3,6-dihydropyridin-1(2H)-yl]sulfonyl}methyl)-2,4-dihydro-3H-1,2,4-triazol-3-one

APCI-MS m/z: 387.1 [MH⁺].
¹H-NMR (DMSO-D6): δ 11.59 (2H, s), 8.72 (2H, s), 6.27 (1H, m), 4.37 (2H, s), 3.90 (2H, brm), 3.35 (2H, brt), 2.35 (2H, brm), 2.21 (1H, m), 1,10 (2H, m), 1.02 (2H, m) ppm.
¹³C-NMR (DMSO-D6): δ 169.69, 158.27, 155.62, 137.61, 131.45, 117.72, 114.93, 94.42, 82.47, 47.48, 44.56, 41.84, 28.80, 18.18, 11.15 ppm.

### Example 8

### 5-({[4-(4-Chlorophenyl)piperidin-1-yl]sulfonyl}methyl)-2,4-dihydro-3H-1,2,4-triazol-3-one

APCI-MS m/z: 357.1 [MH⁺].
¹H-NMR (DMSO-D6): δ 11.60 (1H, s), 11.58 (1H, s), 7.36 (2H, d), 7.29 (2H, d), 4.32 (2H, s), 3.70 (2H, m), 2.93 (2H, m), 2.64 (1H, m), 1.81 (2H, m), 1.59 (2H, m) ppm.

### Example 9

### N-Benzyl-1-(5-oxo-4,5-dihydro-1H-1,2,4-triazol-3-yl)methanesulfonamide

APCI-MS m/z: 269.2 [MH⁺].
¹H-NMR (DMSO-D6): δ 11.54 (1H, s), 11.50 (1H, s), 8.00 (1H, t), 7.38-7.22 (5H, m), 4.21 (2H, s), 4.17 (2H, d) ppm.

### Example 10

### 1-(5-Oxo-4,5-dihydro-1H-1,2,4-tiazol-3-yl)-N-(2-phenylethyl)methanesulfonamide

APCI-MS m/z: 283.2 [MH⁺].
¹H-NMR (DMSO-D6): δ 11.52 (1H, s), 11.46 (1H, s), 7.57 (1H, t), 7.33-7.26 (2H, m), 7.25-7.18 (3H, m), 4.16 (2H, s), 3.17 (2H, q), 2.75 (2H, t) ppm.

Following the general method of Example 2 but substituting the appropriate amine intermediate, and using 1 extra equivalent of the base DIEA if the amine salt was used, the compounds of Examples 11 and 12 were prepared:

### Example 11

### 5-(2-{[4-(4-Chlorophenyl)piperidin-1-yl]sulfonyl}ethyl)-2,4-dihydro-3H-1,2,4-triazol-3-one

APCI-MS m/z: 371.2 [MH⁺].
¹H-NMR (DMSO-D6): δ 11.26 (1H, s), 11.24 (1H, s), 7.36 (2H, d), 7.29 (2H, d), 3.70 (2H, m), 3.39 (2H, t), 2.90 (2H, m), 2.82 (2H, t), 2.66 (1H, m), 1.83 (2H, m), 1.59 (2H, m) ppm.

### Example 12

### 5-(2-{[4-(4-Chlorophenyl)piperazin-1-yl]sulfonyl}ethyl)-2,4-dihydro-3H-1,2,4-triazol-3-one

APCI-MS m/z: 372.2 [MH⁺].
¹H-NMR (DMSO-D6): δ 11.24 (1H, s), 11.22 (1H, s), 7.26 (2H, d), 6.98 (2H, d), 3.42 (2H, t), 3.30 (4H, m), 3.20 (4H, m), 2.82 (2H, t) ppm.

Following the general method of Example 3 but substituting the appropriate amine intermediate, and using 1 extra equivalent of the base DIEA if the amine salt was used, the compounds of Examples 13 and 14 were prepared:

### Example 13

### 5-(3-{[4-(4-Chlorophenyl)piperidin-1-yl]sulfonyl}propyl)-2,4-dihydro-3H-1,2,4-triazol-3-one

APCI-MS m/z: 385.3 [MH⁺].
¹H-NMR (DMSO-D6): δ 11.23 (1H, s), 11.14 (1H, s), 7.36 (2H, d), 7.29 (2H, d), 3.70 (2H, m), 3.13 (2H, t), 2.89 (2H, m), 2.67 (1H, m), 2.54 (2H, t), 1.99 (2H, quintet), 1.83 (2H, m), 1.61 (2H, m) ppm.

### Example 14

### 5-(3-{[4-(4-Chlorophenyl)piperazin-1-yl]sulfonyl}propyl)-2,4-dihydro-3H-1,2,4-triazol-3-one

APCI-MS m/z: 386.2 [MH⁺].
¹H-NMR (DMSO-D6): δ 11.22 (1H, s), 11.12 (1H, s), 7.26 (2H, d), 6.98 (2H, d), 3.29 (4H, m), 3.22 (4H, m), 3.16 (2H, t), 2.53 (2H, t), 1.99 (2H, quintet) ppm.

### Preparation of the non-commercially available amine intermediates used for the Examples:

### 5-Chloro-2-(piperidin-4-yloxy)pyridine

Potassium tert-butoxide (202.0 g, 1.8 mol) was dissolved in THF (1.4 L) at room temperature. Powdered 4-hydroxypiperidine (182.0 g, 1.8 mol) was added in one portion. The clear orange solution was stirred for 25 min.
2,5-Dichloropyridine (226.4 g, 1.53 mol) was dissolved in THF (0.7 L) and added dropwise over 1.5 h to the vigorously stirred solution. After approximately 10 min potassium chloride began to precipitate and the temperature increased to approximately + 40 °C. Stirring was continued overnight at room temperature.
The reaction mixture was filtered and the filtrate evaporated to give an orange oil (346 g). The orange oil was dissolved in dichloromethane (3.0 L) and washed with water (3 x 0.5 L). The organic phase was dried (Na₂SO₄), filtered and evaporated to constant weight. The title compound was obtained as a yellow oil that crystallised to a light yellow solid (287 g, 1.35 mol, 88 %).
APCI-MS m/z: 213.0 [MH⁺].
¹H-NMR (CDCl₃) δ: 8.05 (1H, d), 7.50 (1H, dd), 6.66 (1H, d), 5.07 (1H, m), 3.12 (2H, m), 2.77 (2H, m), 2.03 (2H, m), 1.81 (1H, s), 1.63 (2H, m) ppm.
¹³C-NMR (CDCl₃) δ: 161.38, 144.90, 138.40, 123.57, 112.55, 71.60,44.15, 32.32 ppm.

### 2-Methoxy-5-(1,2,3,6-tetrahydropyridin-4-ylethynyl)pyrimidine hydrochloride

### a) tert-Butyl 4-[(trimethylsilyl)ethynyl]-3,6-dihydropyridine-1(2H-carboxylate

The title compound was prepared from N-Boc-piperidin-4-one as described in WO 96/05200.
¹H NMR (CDCl₃) δ 6.05 (1H, s), 3.94 (2H, dd), 3.47 (2H, t), 2.23 (2H, dq), 1.45 (10H, s), 0,15 (8H, s).
GCMS-MS m/z: 223 [M-56].

### b) tert-Butyl4-ethynyl-3,6-dihydropyridine-1(2H)-carboxylate

tert-Butyl 4-[(trimethylsilyl)ethynyl]-3,6-dihydropyridine-1(2H)-carboxylate (2.85 g, 10.2 mmol) and KF (1.80 g, 30.6 mmol) were dissolved in MeOH (100 mL) and stirred overnight at room temperature. Water was added and the mixture was extracted twice with EtOAc. The organic phase was washed with brine and dried over Na₂SO₄, then filtered and evaporated to give crude product as an oil (2.05 g, 97% yield). This material was further purified by flash chromatography on silica gel with heptane/EtOAc (4:1) as eluent. The fraction containing the required product was evaporated to give a yellow oil that solidified when stored in the freezer (1.39 g).
GCMS-MS m/z: 151 [M-56].
¹H NMR (CDCl₃) δ 6.11 (1H, brs), 3.97 (2H, m), 3.50 (2H, t), 2.89 (1H, s), 2.26 (2H, m), 1.47 (9H, s) ppm.

### c) tert-Butyl 4-[(2-methoxypyrimidin-5-yl)ethynyl]-3,6-dihydropyridine-1(2H)-carboxylate

5-Bromo-2-methoxypyrimidine (238 mg, 1.26 mmol), tert-butyl 4-ethynyl-3,6-dihydropyridine-1(2H)-carboxylate (261 mg, 1.26 mmol), diisopropylamine (0.536 mL, 3.78 mmol) and PdCl₂(PPh₃)₂ (44 mg, 0.06 mmol) were mixed and heated on a oil bath to + 70 °C for 10 minutes. The reaction mixture was treated with water and extracted twice with EtOAc. The combined extracts were dried over Na₂SO₄, filtered and evaporated. The crude product was purified by flash chromatography on silica gel with EtOAc/heptane (3:16) as eluent. Fractions containing the required product were evaporated to give the subtitle compound (179 mg, 45%).
APCI-MS m/z: 316.1 [MH⁺].
¹H-NMR (CDCl₃) δ: 8.56 (2H, s), 6.16 (1H, m), 4.04 (3H+2H, s+m), 3.58 (2H, t), 2.35 (2H, m), 1.49 (9H, s) ppm.

### d) 2-Methoxy-5-(1,2,3,6-tetrahydropyridin-4-ylethynyl)pyrimidine hydrochloride

tert-Butyl 4-[(2-methoxypyrimidin-5-yl)ethynyl]-3,6-dihydropyridine-1 (2H)-carboxylate (179 mg, 0.57 mmol) was dissolved in MeOH (10 mL). 1.8M Hydrogen chloride in tert-butylmethylether (5 mL) was added and the solution was heated to reflux for 1.5 h. The solvents were removed by evaporation and the residual material was dissolved in boiling absolute EtOH. Ether was added and the solution cooled on ice. The precipitate was removed by filtration and washed with EtOH and ether to give the title compound as a slightly yellow solid (82 mg, 57%). The filtrates were evaporated to dryness to give further material (49 mg, 34%) that was slightly more yellow in colour but was pure enough for further use.
APCI-MS m/z: 216.1 [MH⁺].
¹H-NMR (CD₃OD) δ: 8.34 (2H, s), 6.23 (1H, m), 4.03 (3H, s), 3.83 (2H, m), 3.40 (2H, t), 2.61 (2H, m) ppm.

### 5-(1,2,3,6-Tetrahydropyridin-4-ylethynyl)-2-(trifluoromethyl)pyrimidine hydrochloride

### a) 2-(Trifluoromethyl)pyrimidine-5-yl trifluoromethanesulfonate

Triflic anhydride (1.01 mL, 6.0 mmol) was added dropwise to a stirred mixture of 2-(trifluoromethyl)pyrimidin-5-ol (prepared according to US Patent 4,558,039) (0.82 g, 5.0 mmol), toluene (10 mL) and aqueous tripotassium phosphate (30% by weight, 10 mL) at ice-bath temperature (Frantz et al., Organic Letters 2002, 4(26), 4717-4718). When the addition was complete the ice-bath was taken away and the solution was stirred at ambient temperature for 30 minutes. The clear phases were separated and the organic layer was washed with water, then brine. Drying of the organic phase over anhydrous sodium sulfate, filtration and concentration by rotary evaporation at room temperature afforded 1.38 g (93%) of 2-(trifluoromethyl)-pyrimidine-5-yl trifluoromethanesulfonate as a colourless oil.
B.p. 75-77 °C (10 mbar).
¹H NMR (CDCl₃) δ 8.90 (2H, s).

### b) tert-Butyl4-{[2-(trifluoromethyl)pyrimidin-5-yl]ethynyl}-3,6-dihydropyridine-1(2H)-carboxylate

2-(Trifluoromethyl)pyrimidine-5-yl trifluoromethanesulfonate and tert-butyl 4-ethynyl-3,6-dihydropyridine-1(2H)-carboxylate were coupled together in diisopropylamine with PdCl₂(PPh₃)₂ as catalyst as described above in the synthesis of 2-methoxy-5-(1,2,3,6-tetrahydropyridin-4-ylethynyl)pyrimidine hydrochloride.
APCI-MS m/z: 354.1 [MH⁺].
¹H NMR (CDCl₃) δ 8.88 (2H, s), 6.30 (1H, m), 4.08 (2H, dd), 3.58 (2H, t), 2.37 (2H, m), 1.49 (9H, s) ppm.

### c) 5-(1,2,3,6-Tetrahydropyridin-4-ylethynyl)-2-(trifluoromethyl)pyrimidine hydrochloride

Acetyl chloride (0.21 mL, 3 mmol) was added to a cold solution of dry MeOH (10 mL) under argon to form a HCl/MeOH solution. To this solution was added tert-butyl 4-{[2-(trifluoromethyl)pyrimidin-5-yl]ethynyl}-3,6-dihydropyridine-1(2H)-carboxylate (0.353 g, 1 mmol) in portions and the resulting solution was heated to 50 °C for 270 min until deprotection was complete. Evaporation of the solvents gave the subtitle compound in quantitative yield and pure enough for further use.
For analytical purposes, the salt (0.2 g) was recrystallised from MeOH/tert-butyl methyl ether to give a beige coloured solid (0.1 g).
APCI-MS m/z: 254.1 [MH⁺].
¹H NMR (CD₃OD) δ 9.02 (2H, s), 6.38 (1H, m), 3.86 (2H, dd), 3.41 (2H, t), 2.65 (2H, m) ppm.

### 2-Cyclopropyl-5-(1,2,3,6-tetrahydropyridin-4-ylethynyl)pyrimidine trifluoroacetate

### a) 5-(Benzyloxy)-2-cyclopropylpyrimidine

The title compound was prepared following a procedure described in US 4,558,039 using the tetrafluoroborate of Arnold's salt (N-(2-benzyloxy-3-(dimethylamino)-2-propenylidene)-N-methylmethanaminium tetrafluoroborate - Holy, A., Arnold, Z, Collect. Czech. Chem. Commun., EN; 38; 1973; 1371-1380*).*
Cyclopropanecarboxamidine hydrochloride (2.0 g, 16.6 mmol) was dissolved in MeOH (10 mL). To this solution was added Arnold's salt (5.85 g, 18.3 mmol). A solution of NaOMe (2.15 g, 39.8 mmol) in MeOH (20 mL) was added in small portions and the reaction mixture was heated under argon to reflux temperature. After 3.5 h, the reaction mixture was allowed to cool to room temperature and the solvents were removed by evaporation. The solid material was washed with water, filtered off and dried under reduced pressure to give the subtitle compound (2.4 g, 64%).
APCI-MS m/z: 227.1 [MH⁺].
¹H-NMR (DMSO-D₆): δ 8.44 (2H, s), 7.49-7.29 (5H, m), 5.21 (2H, s), 2.14 (1H, m), 0.95 (2H, m), 0.89 (2H, m) ppm.

### b) 2-Cyclopropylpyrimidin-5-ol

5-(Benzyloxy)-2-cyclopropylpyrimidine (3.4 g, 14.9 mmol) in MeOH (40 mL) with 10% Pd on carbon (0.15 g) was hydrogenated at room temperature and 1 atmosphere H₂ (g) pressure for 1.5 h. The mixture was filtered through celite and evaporated to give the subtitle compound as a slightly yellow solid that was pure enough for further use (2.0 g, 100%).
APCI-MS m/z: 137.1 [MH⁺].
¹H-NMR (DMSO-D₆): δ 10.03 (1H, brs), 8.18 (2H, s), 2.09 (1H, m), 0.91 (2H, m), 0.85 (2H, m) ppm.

### c) 2-Cyclopropylpyrimidin-5-yl trifluoromethanesulfonate

2-Cyclopropylpyrimidin-5-ol (1.7 g, 12.5 mmol) was partly dissolved in a mixture of DCM (50 mL) and THF (8 mL). Triethylamine (3.8 g, 37.5 mmol) was added and the cloudy solution was cooled to -15°C. Trifluoromethanesulfonic acid anhydride (5.3 g, 18.7 mmol) dissolved in DCM (10 mL) was slowly added. After 20 minutes, the reaction mixture was transferred to a separation funnel using additional DCM (15 mL), washed with 5% KHCO₃ solution (35 mL) and brine (35 mL). The organic phase was dried over Na₂SO₄, filtered and evaporated to leave the crude product as a black oil. This material was further purified by flash chromatography on silica gel with 40% EtOAc/heptane as eluent to yield the subtitle compound (2.0 g, 62%).
APCI-MS m/z: 269.1 [MH⁺].
¹H-NMR (CDCl₃): δ 8.53 (2H, s), 2.34 (1H, m), 1.20-1.15 (4H, m) ppm.

### d) 2-Cyclopropyl-5-(1,2,3,6-tetrahydropyridin-4-ylethynyl)pyrimidine trifluoroacetate

2-Cyclopropylpyrimidin-5-yl trifluoromethanesulfonate (0.4 g, 1.49 mmol), tert-butyl 4-ethynyl-3,6-dihydropyridine-1(2H)-carboxylate (0.31 g, 1.49 mmol), diethylamine (0.33 g, 4.47 mmol) and PdCl₂(PPh₃)₂ (0.04 g, 0.06 mmol) were placed under argon in a sealed tube and heated to 80 °C for 1.5h. The volatile diethylamine was removed by evaporation and the residual material was dissolved in DCM (10 mL) and treated with TFA (3 mL) at room temperature for 15 minutes. The solvents were removed by evaporation and the residue was purified using a semi-prep HPLC system as follows: KROMASIL 100-5-C18, 250 x 20mm column, UV 220 nm, and a 30 minute gradient of 10 to 90% MeCN/water containing 0.1 % TFA. Fractions containing the required product were collected and evaporated to remove MeCN. Removal of water residues by freeze drying gave the title trifluoroacetic acid salt (50 mg, 10%).
APCI-MS m/z: 226.1 [MH⁺].
¹H-NMR (DMSO-D6): δ 8.85 (2H, brs), 8.74 (2H, s), 6.25 (1H, m), 3.74 (2H, m), 3.26 (2H, t), 2.46 (2H, m), 2.22 (1H, m), 1,11 (2H, m), 1.02 (2H, m) ppm.

### Pharmacological Example

### Isolated Enzyme Assays

Recombinant human MMP12 catalytic domain may be expressed and purified as described by Parkar A.A. et al, (2000), Protein Expression and Purification, 20:152. The purified enzyme can be used to monitor inhibitors of activity as follows: MMP12 (50 ng/ml final concentration) is incubated for 60 minutes at room temperature with the synthetic substrate Mac-Pro-Cha-Gly-Nva-His-Ala-Dpa-NH₂ in assay buffer (0.1M "Tris-HCl" (trade mark) buffer, pH 7.3 containing 0.1M NaCl, 20mM CaCl₂, 0.020 mM ZnCl and 0.05% (w/v) "Brij 35" (trade mark) detergent) in the presence (5 concentrations) or absence of inhibitors. Activity is determined by measuring the fluorescence at λex 320nm and λem 405nm. Percent inhibition is calculated as follows: % Inhibition is equal to the [Fluorescence*_{plus inhibitor} -* Fluorescence*_{background}*] divided by the [Fluorescence,*_{minus inhibiror}*- Fluorescence*_{background}*]

A protocol for testing against other matrix metalloproteinases, including MMP9, using expressed and purified pro MMP is described, for instance, by C. Graham Knight et al., (1992) FEBS Lett. 296(3):263-266.

The following Table shows the IC₅₀ figures (in nanomolar) for a representative selection of the compounds of the Examples when tested against various MMPs.

| **Compound of Example No.** | **Human MMP12 IC₅₀ (nM)** | **Human MMP9 IC₅₀ (nM)** | **Human MMP2 IC₅₀ (nM)** | **Human MMP19 IC₅₀ (nM)** | **Human MMP14 IC₅₀ (nM)** | **Human MMP8 IC₅₀ (nM)** |
|---|---|---|---|---|---|---|
| 2 | 65 | 318 | 1010 | >10000 | 6660 | 243 |
| 4 | 18 | 414 | 142 | 64 | 1750 | 31 |
| 6 | 2.4 | 5.7 | 263 | 4300 | 6850 | 284 |

## Claims

1. A compound of formula (I) or a pharmaceutically acceptable salt or solvate thereof 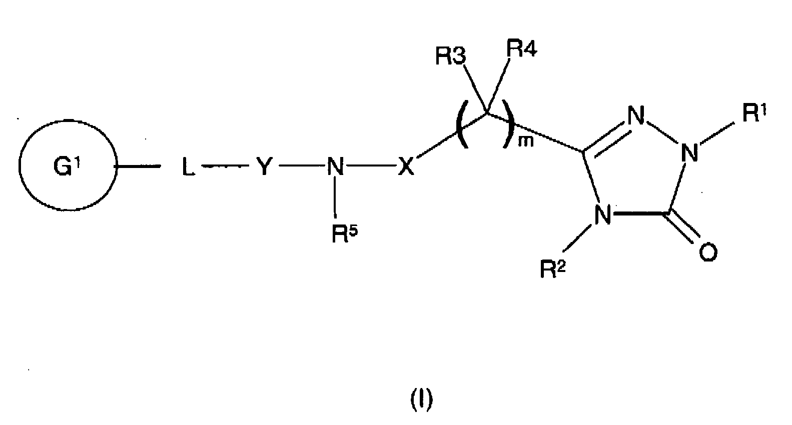 wherein
R¹ and R² independently represent H or C1 to 6 alkyl; said alkyl being optionally further substituted by an aryl ring or an aromatic heterocyclic ring containing 1 to 3 heteroatoms independently selected from O, S and N; said aromatic ring being optionally further substituted by halogen, CF₃, C1 to 4 alkyl or C1 to 4 alkoxy;
Each R³ and each R⁴ independently represents H or C1 to 6 alkyl; said alkyl being optionally further substituted by OH, C1 to 4 alkoxy, C1 to 4 alkylthio, amino, N-alkylamino or N,N-dialkylamino;
or R³ and R⁴ are bonded together so as to form a 3 to 7 membered ring; said ring optionally incorporating one heteroatom selected from O, S(O)q and N;
m represents an integer 1, 2 or 3;
X represents a group S(O), S(O)₂ or C(=O);
R⁵ represents H or C1 to 6 alkyl; said alkyl being optionally further substituted by halogen, OH or C1 to 6 alkoxy;
Y represents a direct bond;
or Y and R⁵ are bonded together such that the group -NR⁵Y- together represents a 4 to 7 membered saturated or partially unsaturated azacyclic ring; said azacyclic ring optionally incorporating one further heteroatom selected from O, S(O)ₙ and N; said azacyclic ring being optionally benzo fused; said azacyclic ring being optionally substituted by C1 to 6 alkyl, C1 to 6 alkoxy or OH;
L represents a direct bond;
or L represents O, S(O)ₚ, C(O), NR⁶, C(O)NR⁶, NR⁶C(O), C2 to 6 alkynyl, C2 to 6 alkenyl, C1 to 6 alkyl, C1 to 6 heteroalkyl or C3 to 6 heteroalkynyl; said alkyl, alkenyl or alkynyl group being optionally further substituted by halogen, OH or C 1 to 6 alkoxy;
n, p and q independently represent an integer 0, 1 or 2;
G¹ represents a monocyclic, bicyclic, tricyclic or tetracyclic group comprising one, two, three or four ring structures each of up to 7 ring atoms; each ring structure being independently selected from cycloalkyl; cycloalkenyl; heterocycloalkyl; unsaturated heterocycloalkyl; aryl; or an aromatic heterocyclic ring containing 1 to 3 heteroatoms independently selected from O, S and N; with each ring structure being independently optionally substituted by one or more substituents independently selected from halogen, hydroxy, CHO, C1 to 6 alkyl, C1 to 6 alkoxy, halo-C1 to 6 alkoxy, amino, N-alkylamino, N,N-dialkylamino, alkylsulfonamino, C2 to 6 alkanoylamino, cyano, nitro, thiol, alkylthio, alkylsulfonyl, alkylaminosulfonyl, C2 to 6 alkanoyl, aminocarbonyl, N-alkylaminocarbonyl, N,N-amino-carbonyl;
wherein any alkyl radical within any substituent may itself be optionally substituted with one or more groups selected from halogen, hydroxy, C1 to 6 alkoxy, halo-C1 to 6 alkoxy, amino, N-alkylamino, N,N-dialkylamino, N-alkylsulfonamino, N-C2 to 6 alkanoylamino, cyano, nitro, thiol, alkylthio, alkylsulfonyl, N-alkylaminosulfonyl, CHO, C2 to 6 alkanoyl, aminocarbonyl. N-alkylaminocarbonyl, N,N-dialkylaminocarbonyl and carbamate; and wherein any alkyl radical is a C 1 to 6 alkyl radical;
and when G¹ is a bicyclic, tricyclic or tetracyclic group, each ring structure is independently joined to the next ring structure by a direct bond, by -O-, by C1-6 alkyl, by C1-6 haloalkyl, by C1-6 heteroalkyl, by C2-6 alkenyl, by C2-6 alkynyl, by sulfone, by CO, by NR⁷CO, by CONR⁷, by NR⁷, by S, or by C(OH), or each ring structure is fused to the next ring structure;
R⁶ and R⁷ independently represent H or C1 to 6 alkyl;
and when the group -NR⁵Y- represents an azacyclic ring and L represents a direct bond, the group G¹ may also be spiro fused to the azacyclic ring;

2. A compound according to claim 1, wherein X represents S(O)₂.

3. A compound according to claim 1 or 2, wherein R¹ and R² each represent hydrogen.

4. A compound according to any one of claims 1 to 3, wherein R³ and R⁴ each represent hydrogen.

5. A compound according to any one of claims 1 to 4, wherein R⁵ represents hydrogen or C1 to 6 alkyl and Y represents a direct bond.

6. A compound according to any one of claims 1 to 4, wherein the group -NR⁵Y-together represents a five or six membered saturated or partially unsaturated azacyclic ring, said azacyclic ring optionally incorporating one further heteroatom selected from O, S(O)ₙ and N.

7. A compound according to any one of claims 1 to 6 wherein L represents a direct bond, O, C2 to 6 alkynyl, G1 to 6 alkyl, C1 to 6 heteroalkyl or C3 to 6 heteroalkynyl.

8. A compound according to any one of claims 1 to 7. wherein G¹ represents an optionally substituted monocyclic or bicyclic ring structure.

9. A compound according to claim 1 which is selected from the group consisting of:
5-[({4-[(5-chloropyridin-2-yl)oxy]piperidin-1-yl}sulfonyl)methyl]-2,4-dihydro-3H-1,2,4-triazol-3-one;
5-[2-({4-[(5-chloropyridin-2-yl)oxy]piperidin-1-yl}sulfonyl)ethyl]-2,4-dihydro-3H-1,2,4-triazol-3-one;
5-[3-({4-[(5-chloropyridin-2-yl)oxy]piperidin-1-yl}sulfonyl)propyl]-2,4-dihydro-3H-1,2,4-triazol-3-one;
5-({[4-(4-chlorophenyl)piperazin-1-yl]sulfonyl}methyl)-2,4-dihydro-3H-1,2,4-triazol-3-one;
5-({[4-[(2-methoxypyrimidin-5-yl)ethynyl]-3,6-dihydropyridin-1(2H)-yl]sulfonyl}methyl)-2,4-dihydro-3H-1,2,4-triazol-3-one;
5-({[4-{[2-(trifluoromethyl)pyrimidin-5-yl]ethynyl}-3,6-dihydropyridin-1(2H)-yl]sulfonyl}methyl)-2,4-dihydro-3H-1,2,4-triazol-3-one;
5-({[4-[(2-cyclopropylpyrimidin-5-yl)ethynyl]-3,6-dihydropyridin-1(2H)-yl]sulfonyl}methyl)-2,4-dihydro-3H-1,2,4-triazol-3-one;
5-({[4-(4-chlorophenyl)piperidin-1-yl]sulfonyl}methyl)-2,4-dihydro-3H-1,2,4-triazol-3-one;
N-benzyl-1-(5-oxo-4,5-dihydro-1H-1,2,4-triazol-3-yl)methanesulfonamide;
1-(5-oxo-4, 5-dihydro-1H-1,2,4-triazol-3-yl)-N-(2-phenylethyl)methanesulfonamide;
5-(2-{[4-(4-chlorophenyl)piperidin-1-yl]sulfonyl}ethyl)-2,4-dihydro-3H-1,2,4-triazol-3-one;
5-(2-{[4-(4-chlorophenyl)piperazin-1-yl]sulfonyl}ethyl)-2,4-dihydro-3H-1,2,4-triazol-3-one;
5-(3-{[4-(4-chlorophenyl)piperidin-1-yl]sulfonyl}propyl)-2,4-dihydro-3H-1,2,4-triazol-3-one;
5-(3-{[4-(4-chlorophenyl)piperazin-1-yl]sulfonyl}propyl)-2,4-dihydro-3H-1,2,4-triazol-3-one;
and pharmaceutically acceptable salts and solvates thereof.

10. A process for the preparation of a compound of formula (I) or a pharmaceutically acceptable salt or solvate thereof as defined in claim 1 which comprises:
reaction of a compound of formula (II) 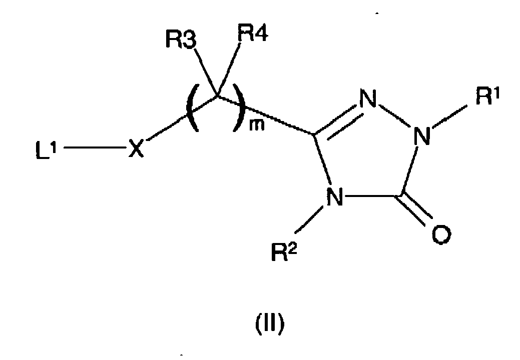 wherein R¹, R², R³, R⁴, X and m are as defined in Claim 1 and L¹ represents a leaving group, with a compound of formula (III) 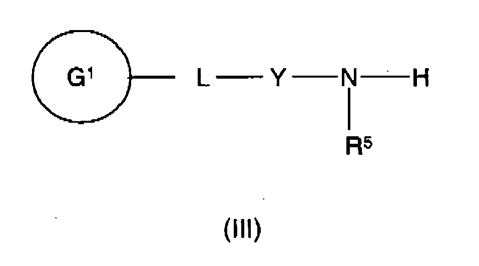
wherein G¹, L, Y and R⁵ are as defined in Claim 1;
and optionally thereafter forming a pharmaceutically acceptable salt or solvate.

11. A pharmaceutical composition comprising a compound of formula (I) or a pharmaceutically acceptable salt or solvate thereof as claimed in any one of claims 1 to 9 in association with a pharmaceutically acceptable adjuvant, diluent or carrier.

12. A process for the preparation of a pharmaceutical composition as claimed in claim 11 which comprises mixing a compound of formula (I) or a pharmaceutically acceptable salt or solvate thereof as defined in any one of claims 1 to 9 with a pharmaceutically acceptable adjuvant, diluent or carrier.

13. A compound of formula (I) or a pharmaceutically acceptable salt or solvate thereof as claimed in any one of claims 1 to 9 for use in therapy.

14. Use of a compound of formula (I) or a pharmaceutically acceptable salt or solvate thereof as claimed in any one of claims 1 to 9 in the manufacture of a medicament for use in the treatment of an obstructive airways disease.

15. Use according to claim 14, wherein the obstructive airways disease is asthma or chronic obstructive pulmonary disease.

## Patentansprüche

1. Verbindungen der Formel (I) und deren pharmazeutisch annehmbare Salze und Solvate, 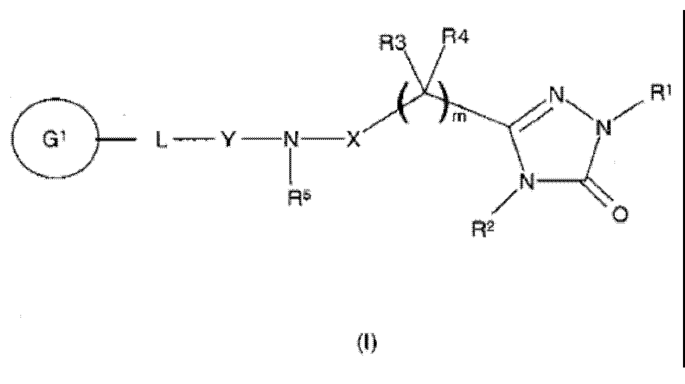 wobei
R¹ und R² unabhängig voneinander für H oder C₁₋₆-Alkyl stehen; wobei Alkyl gegebenenfalls weiter substituiert ist durch einen Arylring oder einen aromatischen heterocyclischen Ring mit 1 bis 3 Heteroatomen unabhängig voneinander ausgewählt aus O, S und N; wobei der aromatische Ring gegebenenfalls weiter substituiert ist durch Halogen, CF₃, C₁₋₄-Alkyl oder C₁₋₄-Alkoxy;
R³ und R⁴ jeweils unabhängig voneinander für H oder C₁₋₆-Alkyl stehen; wobei Alkyl gegebenenfalls weiter substituiert ist durch OH, C₁₋₄-Alkoxy, C₁₋₄-Alkylthio, Amino, N-Alkylamino oder N,N-Dialkylamino;
oder R³ und R⁴ unter Bildung eines 3- bis 7-gliedrigen Rings aneinander gebunden sind; wobei der Ring gegebenenfalls ein Heteroatom ausgewählt aus 0, S(O)_{q} und N enthält;
m für eine ganze Zahl 1, 2 oder 3 steht;
X für eine Gruppe S(O), S(O)₂ oder C(=O) steht;
R⁵ für H oder C₁₋₆-Alkyl steht; wobei Alkyl gegebenenfalls weiter substituiert ist durch Halogen, OH oder C₁₋₆-Alkoxy;
Y für eine direkte Bindung steht;
oder Y und R⁵ aneinander gebunden sind, so daß die Gruppe -NR⁵Y- zusammen für einen 4- bis 7-gliedrigen gesättigten oder teilweise ungesättigten azacyclischen Ring steht; wobei der azacyclische Ring gegebenenfalls ein weiteres Heteroatom ausgewählt aus 0, S(O)ₙ und N enthält;
wobei der azacyclische Ring gegebenenfalls benzokondensiert ist; wobei der azacyclische Ring gegebenenfalls substituiert ist durch C₁₋₆-Alkyl, C₁₋₆-Alkoxy oder OH;
L für eine direkte Bindung steht;
oder L für 0, S(O)ₚ, C(O), NR⁶, C(O)NR⁶, NR⁶C(O), C₂₋₆-Alkinyl, C₂₋₆-Alkenyl, C₁₋₆-Alkyl, C₁₋₆-Heteroalkyl oder C₃₋₆-Heteroalkinyl steht; wobei die Alkyl-, Alkenyl- bzw. Alkinylgruppe gegebenenfalls weiter substituiert ist durch Halogen, OH oder C₁₋₆-Alkoxy;
n, p und q unabhängig voneinander für eine ganze Zahl 0, 1 oder 2 stehen;
G¹ für eine monocyclische, bicyclische, tricyclische oder tetracyclische Gruppe mit einer, zwei, drei oder vier Ringstrukturen mit jeweils bis zu 7 Ringatomen steht; wobei die Ringstrukturen jeweils unabhängig voneinander ausgewählt sind aus Cycloalkyl; Cycloalkenyl; Heterocycloalkyl; ungesättigtem Heterocycloalkyl; Aryl; oder einem aromatischen heterocyclischen Ring mit 1 bis 3 Heteroatomen unabhängig voneinander ausgewählt aus 0, S und N; wobei die Ringstruktur jeweils unabhängig gegebenenfalls substituiert ist durch einen oder mehrere Substituenten unabhängig voneinander ausgewählt aus Halogen, Hydroxy, CHO, C₁₋₆-Alkyl, C₁₋₆-Alkoxy, Halogen-C₁₋₆-alkoxy, Amino, N-Alkylamino, N,N-Dialkylamino, Alkylsulfonamino, C₂₋₆-Alkanoylamino, Cyano, Nitro, Thiol, Alkylthio, Alkylsulfonyl, Alkylaminosulfonyl, C₂₋₆-Alkanoyl, Aminocarbonyl, N-Alkylaminocarbonyl, N,N-Aminocarbonyl;
wobei die Alkylreste in einem Substituenten ihrerseits gegebenenfalls substituiert sein können durch eine oder mehrere Gruppen ausgewählt aus Halogen, Hydroxy, C₁₋₆-Alkoxy, Halogen-C₁₋₆-Alkoxy, Amino, N-Alkylamino, N,N-Dialkylamino, N-Alkylsulfonamino, N-C₂₋₆-Alkanoylamino, Cyano, Nitro, Thiol, Alkylthio, Alkylsulfonyl, N-Alkylaminosulfonyl, CHO, C₂₋₆-Alkanoyl, Aminocarbonyl, N-Alkylaminocarbonyl, N,N-Dialkylaminocarbonyl und Carbamat;
und wobei die Alkylreste C₁₋₆-Alkylreste sind;
und wobei, wenn G¹ für eine bicyclische, tricyclische oder tetracyclische Gruppe steht, die Ringstrukturen jeweils unabhängig voneinander über eine direkte Bindung, über -O-, über C₁₋₆-Alkyl, über C₁₋₆-Halogenalkyl, über C₁₋₆-Heteroalkyl, über C₂₋₆-Alkenyl, über C₂₋₆-Alkinyl, über Sulfon, über CO, über NR⁷CO, über CONR⁷, über NR⁷, über S oder über C(OH) an die nächste Ringstruktur gebunden sind, oder die Ringstrukturen jeweils an die nächste Ringstruktur kondensiert sind;
R⁶ und R⁷ unabhängig voneinander für H oder C₁₋₆-Alkyl stehen;
und wobei, wenn die Gruppe -NR⁵Y- für einen azacyclischen Ring steht und L für eine direkte Bindung steht, die Gruppe G¹ auch an den azacyclischen Ring spiro-kondensiert sein kann.

2. Verbindungen nach Anspruch 1, wobei X für S(O)₂ steht.

3. Verbindungen nach Anspruch 1 oder 2, wobei R¹ und R² jeweils für Wasserstoff stehen.

4. Verbindungen nach einem der Ansprüche 1 bis 3, wobei R³ und R⁴ jeweils für Wasserstoff stehen.

5. Verbindungen nach einem der Ansprüche 1 bis 4, wobei R⁵ für Wasserstoff oder C₁₋₆-Alkyl steht und Y für eine direkte Bindung steht.

6. Verbindungen nach einem der Ansprüche 1 bis 4, wobei die Gruppe -NR⁵Y- zusammen für einen 5- oder 6-gliedrigen gesättigten oder teilweise ungesättigten azacyclischen Ring steht, wobei der azacyclische Ring gegebenenfalls ein weiteres Heteroatom ausgewählt aus 0, S(O)ₙ und N enthält.

7. Verbindungen nach einem der Ansprüche 1 bis 6, wobei L für eine direkte Bindung, 0, C₂₋₆-Alkinyl, C₁₋₆-Alkyl, C₁₋₆-Heteroalkyl oder C₃₋₆-Heteroalkinyl steht.

8. Verbindungen nach einem der Ansprüche 1 bis 7, wobei G¹ für eine gegebenenfalls substituierte monocyclische oder bicyclische Ringstruktur steht.

9. Verbindungen nach Anspruch 1, ausgewählt aus der Gruppe bestehend aus:
5-[({4-[(5-Chlorpyridin-2-yl)oxy]piperidin-1-yl}-sulfonyl)methyl]-2,4-dihydro-3H-1,2,4-triazol-3-on;
5-[2-({4-[(5-Chlorpyridin-2-yl)oxy]piperidin-1-yl}sulfonyl)ethyl]-2,4-dihydro-3H-1,2,4-triazol-3-on;
5-[3-({4-[(5-Chlorpyridin-2-yl)oxy]piperidin-1-yl}sulfonyl)propyl]-2,4-dihydro-3H-1,2,4-triazol-3-on;
5-[({4-(4-Chlorphenyl)piperazin-1-yl}-sulfonyl)methyl]-2,4-dihydro-3H-1,2,4-triazol-3-on;
5-({[4-[(2-Methoxypyrimidin-5-yl)ethinyl]-3,6-dihydropyridin-1-(2H)-yl]sulfonyl}methyl)-2,4-dihydro-3H-1,2,4-triazol-3-on;
5-({[4-{[2-(Trifluormethyl)pyrimidin-5-yl]ethinyl}-3,6-dihydropyridin-1-(2H)-yl]sulfonyl}methyl)-2,4-dihydro-3H-1,2,4-triazol-3-on;
5-({[4-[(2-Cyclopropylpyrimidin-5-yl)ethinyl]-3,6-dihydropyridin-1-(2H)-yl]sulfonyl}methyl)-2,4-dihydro-3H-1,2,4-triazol-3-on;
5-({[4-(4-Chlorphenyl)piperidin-1-yl]sulfonyl}methyl)-2,4-dihydro-3H-1,2,4-triazol-3-on;
N-Benzyl-1-(5-oxo-4,5-dihydro-1H-1,2,4-triazol-3-yl)methansulfonamid;
1-(5-Oxo-4,5-dihydro-1H-1,2,4-triazol-3-yl)-N-(2-phenylethyl)methansulfonamid;
5-(2-{[4-(4-Chlorphenyl)piperidin-1-yl]sulfonyl}ethyl)-2,4-dihydro-3H-1,2,4-triazol-3-on;
5-(2-{[4-(4-Chlorphenyl)piperazin-1-yl]sulfonyl}ethyl)-2,4-dihydro-3H-1,2,4-triazol-3-on;
5-(3-{[4-(4-Chlorphenyl)piperidin-1-yl]sulfonyl}propyl)-2,4-dihydro-3H-1,2,4-triazol-3-on;
5-(3-{[4-(4-Chlorphenyl)piperazin-1-yl]sulfonyl}propyl)-2,4-dihydro-3H-1,2,4-triazol-3-on;
und deren pharmazeutisch annehmbaren Salzen und Solvaten.

10. Verfahren zur Herstellung einer Verbindung der Formel (I) oder eines pharmazeutisch annehmbaren Salzes oder Solvates davon nach Anspruch 1, bei dem man:
eine Verbindung der Formel (II) 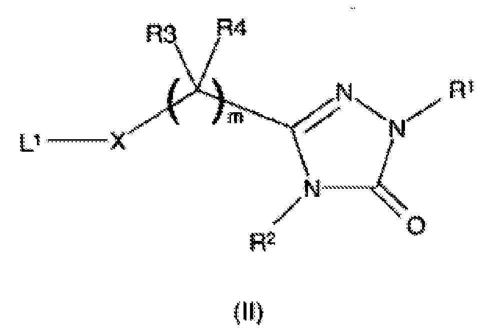 in welcher R¹, R², R³, R⁴, X und m wie in Anspruch 1 definiert sind und L¹ für eine Abgangsgruppe steht, mit einer Verbindung der Formel (III) 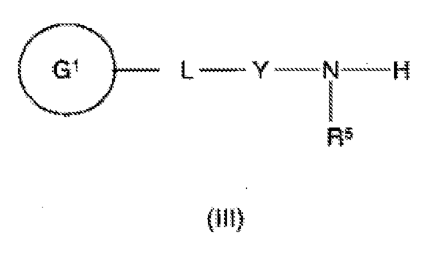
in welcher G¹, L, Y und R⁵ wie in Anspruch 1 definiert sind, umsetzt;
und gegebenenfalls im Anschluß daran ein pharmazeutisch annehmbares Salz oder Solvat bildet.

11. Pharmazeutische Zusammensetzung, enthaltend eine Verbindung der Formel (I) oder ein pharmazeutisch annehmbares Salz oder Solvat davon nach einem der Ansprüche 1 bis 9 zusammen mit einem pharmazeutisch annehmbaren Adjuvans, Verdünnungsmittel oder Träger.

12. Verfahren zur Herstellung einer pharmazeutischen Zusammensetzung nach Anspruch 11, bei dem man eine Verbindung der Formel (I) oder ein pharmazeutisch annehmbares Salz oder Solvat davon nach einem der Ansprüche 1 bis 9 mit einem pharmazeutisch annehmbaren Adjuvans, Verdünnungsmittel oder Träger mischt.

13. Verbindungen der Formel (I) und deren pharmazeutisch annehmbare Salze oder Solvate nach einem der Ansprüche 1 bis 9 zur Verwendung in der Therapie.

14. Verwendung einer Verbindung der Formel (I) oder eines pharmazeutisch annehmbaren Salzes oder Solvates davon nach einem der Ansprüche 1 bis 9 bei der Herstellung eines Medikaments zur Verwendung bei der Behandlung einer obstruktiven Atemwegserkrankung.

15. Verwendung nach Anspruch 14, wobei es sich bei der obstruktiven Atemwegserkrankung um Asthma oder chronische obstruktive Atemwegserkrankung handelt.

## Revendications

1. Composé de formule (I) ou un sel ou solvate pharmaceutiquement acceptable de celui-ci 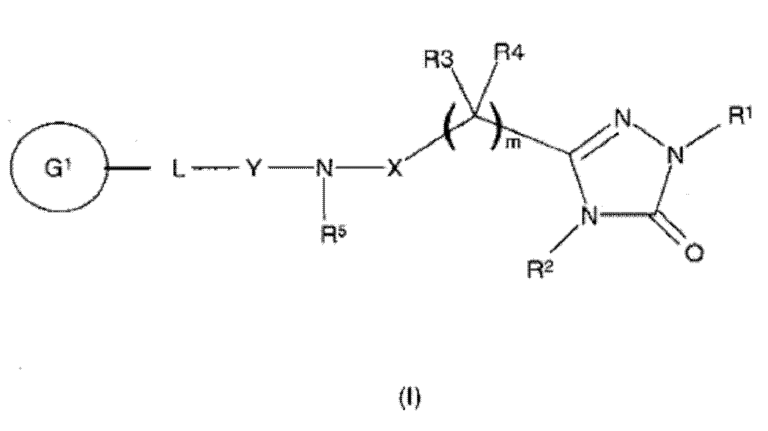 dans laquelle
R¹ et R² représentent indépendamment H ou alkyle en C1 à 6 ; ledit alkyle étant éventuellement encore substitué par un cycle aryle ou un cycle hétérocyclique aromatique contenant de 1 à 3 hétéroatomes choisis indépendamment parmi O, S et N ; ledit cycle aromatique étant éventuellement encore substitué par halogène, CF₃, alkyle en C1 à 4 ou alcoxy en C1 à 4 ;
chaque R³ et chaque R⁴ représentent indépendamment H ou alkyle en C1 à 6 ; ledit alkyle étant éventuellement encore substitué par OH, alcoxy en C1 à 4, alkylthio en C1 à 4, amino, N-alkylamino
ou N,N-dialkylamino ;
ou R³ et R⁴ sont liés ensemble de manière à former un cycle à 3 à 7 chaînons ; ledit cycle comportant éventuellement un hétéroatome choisi parmi O, S(O)_{q} et N ;
m représente un entier 1, 2 ou 3 ;
X représente un groupement S(O), S(O)₂ ou C(=O) ;
R⁵ représente H ou alkyle en C1 à 6 ; ledit alkyle étant éventuellement encore substitué par halogène, OH ou alcoxy en C1 à 6 ;
Y représente une liaison directe ;
ou Y et R⁵ sont liés ensemble de sorte que le groupement -NR⁵Y- représente ensemble un cycle azacyclique saturé ou partiellement insaturé à 4 à 7 chaînons ; ledit cycle azacyclique comportant éventuellement un autre hétéroatome choisi parmi O, S(O)ₙ et N ; ledit cycle azacyclique étant éventuellement condensé sur benzo ; ledit cycle azacyclique étant éventuellement substitué par alkyle en C1 à 6, alcoxy en C1 à 6 ou OH ;
L représente une liaison directe ;
ou L représente O, S(O)ₚ, C(O), NR⁶, C(O)NR⁶, NR⁶C(O), alcynyle en C2 à 6, alcényle en C2 à 6, alkyle en C1 à 6, hétéroalkyle en C1 à 6 ou hétéroalcynyle en C3 à 6 ; ledit groupement alkyle, alcényle ou alcynyle étant éventuellement encore substitué par halogène, OH ou alcoxy en C1 à 6 ;
n, p et q représentent indépendamment un entier 0, 1 ou 2 ;
G¹ représente un groupement monocyclique, bicyclique, tricyclique ou tétracyclique comprenant des structures à un, deux, trois ou quatre cycles dont chacune a jusqu'à 7 atomes du cycle ; chaque structure cyclique étant choisie indépendamment parmi cycloalkyle ; cycloalcényle ; hétérocycloalkyle ; hétérocycloalkyle insaturé ; aryle ; ou un cycle hétérocyclique aromatique contenant de 1 à 3 hétéroatomes choisis indépendamment parmi 0, S et N ; chaque structure cyclique étant éventuellement indépendamment substituée par un ou plusieurs substituants choisis indépendamment parmi halogène, hydroxy, CHO, alkyle en C1 à 6, alcoxy en C1 à 6, halogénoalcoxy en C1 à 6, amino, N-alkylamino, N,N-dialkylamino, alkylsulfonamino, alcanoylamino en C2 à 6, cyano, nitro, thiol, alkylthio, alkylsulfonyle, alkylaminosulfonyle, alcanoyle en C2 à 6, aminocarbonyle, N-alkylaminocarbonyle, N,N-aminocarbonyle ;
où tout radical alkyle dans un substituant quelconque peut lui-même être éventuellement substitué par un ou plusieurs groupements choisis parmi halogène, hydroxy, alcoxy en C1 à 6, halogénoalcoxy en C1 à 6, amino, N-alkylamino, N,N-dialkylamino, N-alkylsulfonamino, N-alcanoylamino en C2 à 6, cyano, nitro, thiol, alkylthio, alkylsulfonyle, N-alkylaminosulfonyle, CHO, alcanoyle en C2 à 6, aminocarbonyle, N-alkylaminocarbonyle, N,N-dialkylaminocarbonyle et carbamate ;
et où tout radical alkyle est un radical alkyle en C1 à 6 ;
et lorsque G¹ est un groupement bicyclique, tricyclique ou tétracyclique, chaque structure cyclique est jointe indépendamment à la structure cyclique suivante par une liaison directe, par -O- , par alkyle en C1-6, par halogénoalkyle en C1-6, par hétéroalkyle en C1-6, par alcényle en C2-6, par alcynyle en C2-6, par sulfone, par CO, par NR⁷CO, par CONR⁷, par NR⁷, par S, ou par C(OH), ou chaque structure cyclique est condensée sur la structure cyclique suivante ;
R⁶ et R⁷ représentent indépendamment H ou alkyle en C1 à 6 ;
et lorsque le groupement -NR⁵Y- représente un cycle azacyclique et L représente une liaison directe, le groupement G¹ peut également être spiro-condensé sur le cycle azacyclique.

2. Composé selon la revendication 1, **caractérisé en ce que** X représente S(O)₂.

3. Composé selon la revendication 1 ou 2, **caractérisé en ce que** R¹ et R² représentent chacun hydrogène.

4. Composé selon l'une quelconque des revendications 1 à 3, **caractérisé en ce que** R³ et R⁴ représentent chacun hydrogène.

5. Composé selon l'une quelconque des revendications 1 à 4, **caractérisé en ce que** R⁵ représente hydrogène ou alkyle en C1 à 6 et Y représente une liaison directe.

6. Composé selon l'une quelconque des revendications 1 à 4, **caractérisé en ce que** le groupement -NR⁵Y-représente ensemble un cycle azacyclique saturé ou partiellement insaturé à cinq ou six chaînons, ledit cycle azacyclique comportant éventuellement un autre hétéroatome choisi parmi O, S(O)ₙ et N.

7. Composé selon l'une quelconque des revendications 1 à 6, **caractérisé en ce que** L représente une liaison directe, O, alcynyle en C2 à 6, alkyle en C1 à 6, hétéroalkyle en C1 à 6 ou hétéroalcynyle en C3 à 6.

8. Composé selon l'une quelconque des revendications 1 à 7, **caractérisé en ce que** G¹ représente une structure de cycle monocyclique ou bicyclique éventuellement substituée.

9. Composé selon la revendication 1, **caractérisé en ce qu'**il est choisi dans le groupe constitué de :
5-[({4-[(5-chloropyridin-2-yl)oxy]pipéridin-1-yl}sulfonyl)méthyl]-2,4-dihydro-3H-1,2,4-triazol-3-one ;
5-[2-({4-[(5-chloropyridin-2-yl)oxy]pipéridin-1-yl}sulfonyl)éthyl]-2,4-dihydro-3H-1,2,4-triazol-3-one ;
5-[3-({4-[(5-chloropyridin-2-yl)oxy]pipéridin-1-yl}sulfonyl)propyl]-2,4-dihydro-3H-1,2,4-triazol-3-one ;
5-({[4-(4-chlorophényl)pipérazin-1-yl]sulfonyl}méthyl)-2,4-dihydro-3H-1,2,4-triazol-3-one ;
5-({[4-[(2-méthoxypyrimidin-5-yl)éthynyl]-3,6-dihydropyridin-1(2H)yl]sulfonyl}méthyl)-2,4-dihydro-3H-1,2,4-triazol-3-one ;
5-({[4-{[2-(trifluorométhyl)pyrimidin-5-yl]éthynyl}-3,6-dihydropyridin-1(2H)yl]sulfonyl}méthyl)-2,4-dihydro-3H-1,2,4-triazol-3-one ;
5-({[4-[(2-cyclopropylpyrimidin-5-yl)éthynyl]-3,6-dihydropyridin-1(2H)yl]sulfonyl}méthyl)-2,4-dihydro-3H-1,2,4-triazol-3-one ;
5-({[4-(4-chlorophényl)pipéridin-1-yl]sulfonyl}méthyl)-2,4-dihydro-3H-1,2,4-triazol-3-one ;
N-benzyl-1-(5-oxo-4,5-dihydro-1H-1,2,4-triazol-3-yl)méthanesulfonamide ;
1-(5-oxo-4,5-dihydro-1H-1,2,4-triazol-3-yl)-N-(2-phényléthyl)méthanesulfonamide ;
5-(2-{[4-(4-chlorophényl)pipéridin-1-yl]sulfonyl}éthyl)-2,4-dihydro-3H-1,2,4-triazol-3-one ;
5-(2-{[4-(4-chlorophényl)pipérazin-1-yl]sulfonyl}éthyl)-2,4-dihydro-3H-1,2,4-triazol-3-one ;
5-(3-{[4-(4-chlorophényl)pipéridin-1-yl]sulfonyl}propyl)-2,4-dihydro-3H-1,2,4-triazol-3-one ;
5-(3-{[4-(4-chlorophényl)pipérazin-1-yl]sulfonyl}propyl)-2,4-dihydro-3H-1,2,4-triazol-3-one ;
et des sels et solvates pharmaceutiquement acceptables de ceux-ci.

10. Procédé de préparation d'un composé de formule (I) ou d'un sel ou solvate pharmaceutiquement acceptable de celui-ci, tel que défini dans la revendication 1, **caractérisé en ce qu'**il comprend :
la réaction d'un composé de formule (II) 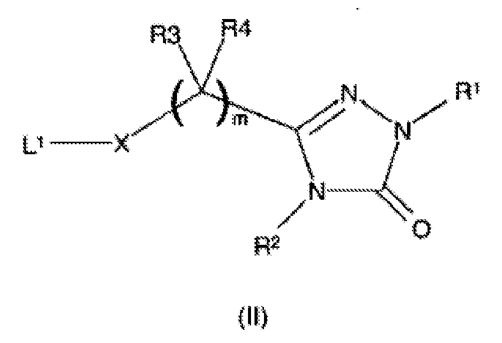 dans laquelle R¹, R², R³, R⁴, X et m sont tels que définis dans la revendication 1 et L¹ représente un groupement partant, avec un composé de formule (III) 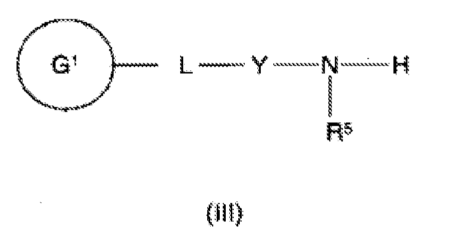
dans laquelle G¹, L, Y et R⁵ sont tels que définis dans la revendication 1 ;
et ensuite éventuellement la formation d'un sel ou solvate pharmaceutiquement acceptable.

11. Composition pharmaceutique comprenant un composé de formule (I) ou un sel ou solvate pharmaceutiquement acceptable de celui-ci selon l'une quelconque des revendications 1 à 9 en association avec un adjuvant, un diluant ou un support pharmaceutiquement acceptable.

12. Procédé de préparation d'une composition pharmaceutique selon la revendication 11, **caractérisé en ce qu'**il comprend le mélange d'un composé de formule (I) ou d'un sel ou solvate pharmaceutiquement acceptable de celui-ci, tel que défini dans l'une quelconque des revendications 1 à 9, avec un adjuvant, un diluant ou un support pharmaceutiquement acceptable.

13. Composé de formule (I) ou un sel ou solvate pharmaceutiquement acceptable de celui-ci selon l'une quelconque des revendications 1 à 9, destiné à être utilisé en thérapie.

14. Utilisation d'un composé de formule (I) ou d'un sel ou solvate pharmaceutiquement acceptable de celui-ci selon l'une quelconque des revendications 1 à 9 dans la fabrication d' un médicament destiné à être utilisé dans le traitement d'une maladie obstructive des voies aériennes.

15. Utilisation selon la revendication 14, **caractérisée en ce que** la maladie obstructive des voies aériennes est l'asthme ou la bronchopneumopathie chronique obstructive.
